# EUROPEAN PATENT APPLICATION

(11) **EP 4 585 593 A1**
(43) Date of publication of application: **16.07.2025**
(21) Application number: 23862443.1
(22) Date of filing: 06.09.2023
(51) Int. Cl.: C07D 405/14, C07D 403/14, C07D 401/14, C07D 487/00, A61K 31/4184, A61K 31/444, A61P 3/00, A61P 5/50, A61P 19/02, A61P 9/10, A61P 43/00

(54) **POLYMORPH OF GLP-1R AGONIST COMPOUND, PREPARATION METHOD THEREFOR, AND USE THEREOF**

(30) Priority: 06.09.2022 CN 202211096444
(71) Applicant: Minidrank Therapeutics (Suzhou) New Drug Research and Development Co., Ltd, Suzhou, Jiangsu 215127 (CN); Mindrank AI Ltd., Hangzhou, Zhejiang 310018 (CN)
(72) Inventor: ZHANG, Long, Hangzhou, China (Zhejiang) Pilot Free Trade Zone Hangzhou, Zhejiang 310018 (CN); NIU, Zhangming, Hangzhou, China (Zhejiang) Pilot Free Trade Zone Hangzhou, Zhejiang 310018 (CN); HU, Yang, Hangzhou, China (Zhejiang) Pilot Free Trade Zone Hangzhou, Zhejiang 310018 (CN)
(74) Representative: Gille Hrabal Partnerschaftsgesellschaft mbB Patentanwälte
(86) International application number: PCT/CN2023/117300
(87) International publication number: WO 2024/051749

(57) **Abstract**

Provided are a polymorph of a GLP-1R agonist compound I, a preparation method therefor, and use thereof. Compared with the free state of the compound I, the polymorph has higher stability and better processability, and is more suitable for preparing drugs for preventing or treating diseases related to a GLP-1R target and a signaling pathway thereof, such as type 2 diabetes, prediabetes, obesity, non-alcoholic fatty liver disease, non-alcoholic steatohepatitis, nephropathy, gout, hyperhematuria, and cardiovascular diseases.

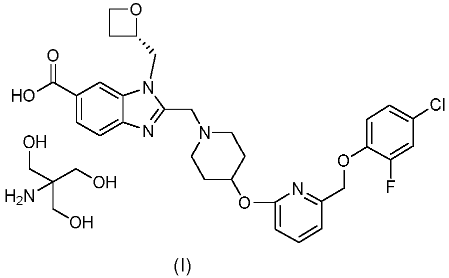

## Description

The present application claims priority to the prior Patent Application No. 2022110964440 entitled "POLYMORPH OF GLP-1R AGONIST COMPOUND, PREPARATION METHOD THEREFOR, AND USE THEREOF" and filed with the China National Intellectual Property Administration on September 6, 2022, the content of which is incorporated herein by reference in its entirety.

### TECHNICAL FIELD

The present disclosure relates to the field of pharmaceutical chemistry, and particularly, to a polymorph of a GLP-1R agonist compound, a method for preparing same, and use thereof.

### BACKGROUND

Diabetes is a chronic disease characterized by high blood glucose levels and is caused by (relatively or absolutely) insufficient insulin secretion or insulin action disorders. According to the ninth edition (the latest edition) of the International Diabetes Federation (IDF) Diabetes Atlas, approximately 463 million adults (aged 20-79) worldwide were living with diabetes in 2019, and the number of patients with diabetes is projected to be 578 million by 2030. At this rate, 700 million people worldwide will be living with diabetes in 2045. Therefore, diabetes has become one of the most pressing global social health problems of the 21st century.

Currently, various pharmacological methods are available to treat hyperglycemia and the attendant T2DM (Hampp et al., Use of Antidiabetic Drugs in the U.S., 2003-2012, Diabetes Care, 37:1367-1374, 2014). These methods can be classified into six major categories, each acting through a different major mechanism.

Insulin secretagogues, including sulfonylureas, dipeptidyl peptidase IV (PP-IV) inhibitors, and glucagon-like peptide-1 receptor (GLP-1R) agonists, enhance insulin secretion by acting on pancreatic β cells. Sulfonylureas have limited efficacy and tolerability, cause weight gain, and often induce hypoglycemia. DP-IV inhibitors have limited efficacy. Commercially available GLP-1R agonists are peptides that are administered by subcutaneous injection, and liraglutide is approved for the treatment of obesity.

Biguanides, such as metformin, are believed to act mainly by reducing hepatic glucose production. Biguanides often cause gastrointestinal discomfort and lactic acidosis, which further limit their use.

α-Glucosidase inhibitors, such as acarbose, can reduce intestinal glucose absorption. These medicaments often cause gastrointestinal discomfort.

Thiazolidinediones, such as pioglitazone and rosiglitazone, act on specific receptors in the liver, muscle, and adipose tissue. They regulate lipid metabolism and subsequently enhance the responses of these tissues to insulin action. Frequent use of these drugs may cause weight gain and may induce edema and anemia.

Insulin, either alone or in combination with the medicaments described above, is used for more severe cases, and frequent use of insulin may also cause weight gain and involve a risk of hypoglycemia.

Sodium-glucose cotransporter 2 (SGLT2) inhibitors (such as dapagliflozin, empagliflozin, canagliflozin, and ertugliflozin) inhibit glucose reabsorption in the kidneys, thereby lowering blood glucose levels. This emerging drug may be associated with ketoacidosis and urinary tract infections.

However, except for GLP-1R agonists and SGLT2 inhibitors, these drugs have limited efficacy and fail to address the most important issues: the functional decline of β cells and related obesity. Therefore, there is a need for more effective pharmaceuticals with relatively few side effects and easy administration.

GLP-1 is a 30-amino-acid-long incretin hormone secreted by intestinal L cells in response to food intake. GLP-1 has been shown to stimulate insulin secretion in a physiological and glucose-dependent manner, reduce glucagon secretion, inhibit gastric emptying, reduce appetite, and stimulate β cell proliferation. In non-clinical trials, GLP-1 promotes continued β cell competence by stimulating the transcription of genes important for glucose-dependent insulin secretion and by promoting β cell neogenesis (Meier, et al., Biodrugs, 17(2):93-102, 2013).

In healthy individuals, GLP-1 plays an important role in regulating postprandial blood glucose levels by stimulating glucose-dependent insulin secretion by the pancreas to increase peripheral glucose absorption. GLP-1 also inhibits glucagon secretion, resulting in reduced hepatic glucose output. In addition, GLP-1 delays gastric emptying and slows small intestine movements to delay food absorption. In people with T2DM, the normal postprandial rise in GLP-1 is absent or reduced (Vilsboll et al., Diabetes, 50:609-613, 2001).

The structure of GLP-1 has been correspondingly engineered and modified in scientific research to increase its half-life and thus prolong its *in vivo* biological effect. However, currently, clinically used long-acting GLP-1 analogs, such as liraglutide, exenatide, etc., are all polypeptides and require frequent injections, which lead to relatively poor patient compliance. Therefore, the development of small-molecule GLP-1R agonists will focus on improving patient compliance, simplifying administration, and reducing side effects of drugs; these agonists have a wide clinical market prospect.

Hangzhou Mindrank AI Ltd. has developed a class of small-molecule compounds with novel structures and potent GLP-1R agonistic activity. Among them, a compound of formula (I) with the chemical name (*S*)-2-((4-((6-((4-chloro-2-fluorophenoxy)methyl)pyridin-2-yl)oxy)piperidin-1-yl)methyl)-1-(oxetan-2-ylmethyl)-1*H*-benzo[d]imidazole-6-carboxylic acid tromethamine salt (1:1) exhibits potent GLP1R receptor agonistic activity and good druggability, and the structure of the compound is as follows:

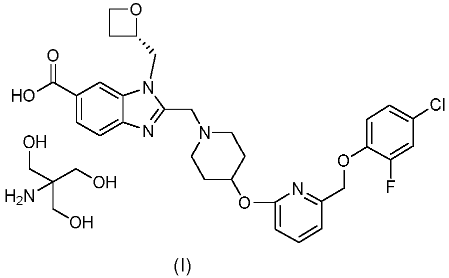

The successful development of solid forms of a drug often requires such properties as a solid form features easy separation and purification after synthesis and a dosage form suitable for industrial production, for long-term preservation with minimized water absorption, decomposition or conversion to other solid forms and for rapid absorption in the human body after administration (e.g., soluble in water and gastric juice). To meet the needs of clinical research and the approval of pharmaceutical formulations, it is imperative to develop a drug crystal form that is easy to separate and purify, suitable for industrial production, and physicochemically stable.

### SUMMARY

To solve the problems with the prior art, in a first aspect of the present disclosure, provided is a polymorph of compound I shown as follows,

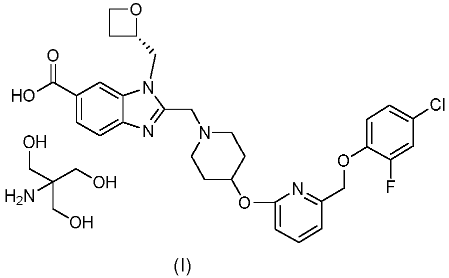

According to the embodiments of the present disclosure, the polymorph is a non-solvate crystal form, a hydrate crystal form, a solvate crystal form, or a metastable crystal form of compound I.

According to the embodiments of the present disclosure, the non-solvate crystal form may be the following crystal form A, B, or C; the hydrate crystal form may be the following crystal form D; the solvate crystal form may be the following crystal form E, F, G, H, or I; and the metastable crystal form may be the following crystal form J, K, L, M, or N.

The present disclosure provides a crystal form A of compound I, which has an X-ray powder diffraction pattern comprising peaks at diffraction angles (2θ) of 7.42±0.2°, 3.68±0.2°, 21.85±0.2°, and 18.75±0.2°.

According to the present disclosure, preferably, the crystal form A further comprises peaks at diffraction angles (2θ) of 9.29±0.2°, 16.71±0.2°, 11.18±0.2°, 15.32±0.2°, and 14.94±0.2°.

According to the present disclosure, more preferably, the crystal form A further comprises peaks at diffraction angles (2θ) of 27.81±0.2°, 17.00±0.2°, 19.81±0.2°, 11.80±0.2°, 25.69±0.2°, and 17.43±0.2°.

Preferably, the crystal form A has an X-ray powder diffraction pattern comprising diffraction angles (2θ) shown in Table 1, wherein the 2θ angles have a margin of error of ±0.20°:

**Table 1**

| 2θ (°) | Intensity% | 2θ (°) | Intensity% |
|---|---|---|---|
| 3.68 | 51.4 | 19.81 | 6.5 |
| 7.42 | 100.0 | 20.94 | 1.9 |
| 9.29 | 13.0 | 21.85 | 27.3 |
| 11.18 | 12.1 | 22.98 | 1.5 |
| 11.80 | 6.3 | 23.80 | 2.2 |
| 14.45 | 4.3 | 25.69 | 5.7 |
| 14.94 | 8.7 | 27.81 | 6.7 |
| 15.32 | 9.3 | 28.73 | 3.4 |
| 15.65 | 3.4 | 30.07 | 2.1 |
| 16.71 | 12.8 | 32.14 | 1.1 |
| 17.00 | 6.5 | 33.43 | 1.5 |
| 17.43 | 4.9 | 33.93 | 2.4 |
| 18.75 | 26.1 | | |

Preferably, the crystal form A has X-ray powder diffraction intensities shown in Table 1.

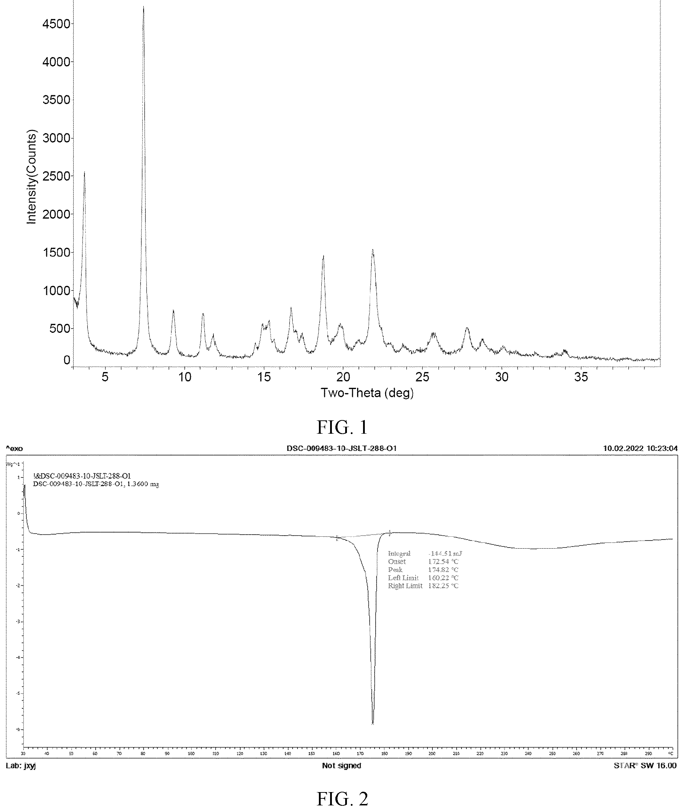

Preferably, the crystal form A has an X-ray powder diffraction pattern substantially as shown in FIG. 1.

Preferably, the crystal form A exhibits an endothermic peak in a DSC analysis when heated to a peak temperature of about 174.82 °C.

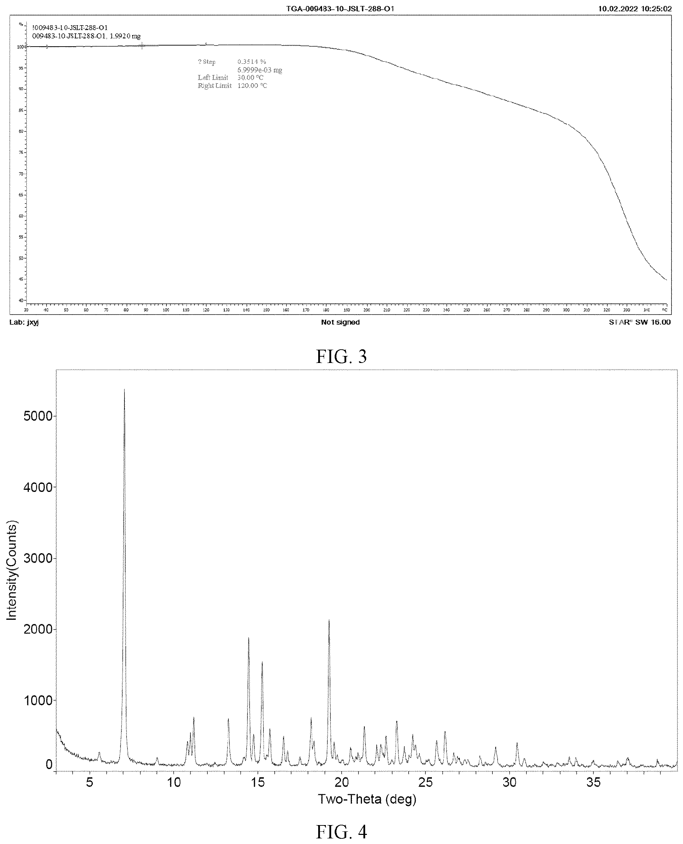

Preferably, the crystal form A has a DSC profile substantially as shown in FIG. 2. Preferably, the crystal form A has a TGA profile substantially as shown in FIG. 3.

The present disclosure provides a crystal form B of compound I, which has an X-ray powder diffraction pattern comprising peaks at diffraction angles (2θ) of 7.07±0.2°, 19.25±0.2°, 14.45±0.2°, and 15.26±0.2°.

According to the present disclosure, preferably, the crystal form B further comprises peaks at diffraction angles (2θ) of 11.20±0.2°, 18.17±0.2°, 13.26±0.2°, 23.28±0.2°, and 21.35±0.2°.

According to the present disclosure, more preferably, the crystal form B further comprises peaks at diffraction angles (2θ) of 15.72±0.2°, 26.17±0.2°, 16.54±0.2°, 24.23±0.2°, 14.76±0.2°, and 22.65±0.2°.

According to the present disclosure, preferably, the crystal form B has an X-ray powder diffraction pattern comprising diffraction angles (2θ) shown in Table 2, wherein the 2θ angles have a margin of error of ±0.20°:

**Table 2**

| 2θ (°) | Intensity% | 2θ (°) | Intensity% |
|---|---|---|---|
| 5.58 | 2.4 | 22.65 | 7.3 |
| 7.07 | 100.0 | 23.28 | 11.3 |
| 9.02 | 2.1 | 23.72 | 4.0 |
| 10.81 | 6.4 | 24.23 | 7.8 |
| 11.20 | 13.0 | 24.62 | 2.7 |
| 12.43 | 0.9 | 25.18 | 1.4 |
| 13.26 | 12.4 | 25.65 | 6.6 |
| 14.18 | 1.9 | 26.17 | 8.9 |
| 14.45 | 33.7 | 26.67 | 3.2 |
| 14.76 | 7.5 | 27.01 | 2.0 |
| 15.26 | 27.0 | 27.33 | 1.6 |
| 15.56 | 2.3 | 27.51 | 1.5 |
| 15.72 | 9.4 | 28.23 | 2.7 |
| 16.54 | 7.8 | 29.18 | 5.1 |
| 16.77 | 4.0 | 30.45 | 6.4 |
| 17.51 | 2.3 | 30.86 | 2.1 |
| 18.17 | 12.7 | 31.50 | 0.7 |
| 18.64 | 0.7 | 32.01 | 1.2 |
| 19.25 | 38.8 | 32.83 | 0.8 |
| 19.54 | 6.1 | 33.56 | 2.7 |
| 19.75 | 2.6 | 33.94 | 2.4 |
| 20.07 | 0.8 | 35.00 | 1.7 |
| 20.53 | 4.4 | 36.43 | 1.4 |
| 20.96 | 3.3 | 37.05 | 2.5 |
| 21.35 | 10.3 | 37.87 | 0.6 |
| 22.09 | 5.3 | 38.79 | 2.0 |
| 22.32 | 5.4 | | |

Preferably, the crystal form B has X-ray powder diffraction intensities shown in Table 2.

Preferably, the crystal form B has an X-ray powder diffraction pattern substantially as shown in FIG. 4.

Preferably, the crystal form B exhibits an endothermic peak in a DSC analysis when heated to a peak temperature of about 160.15 °C.

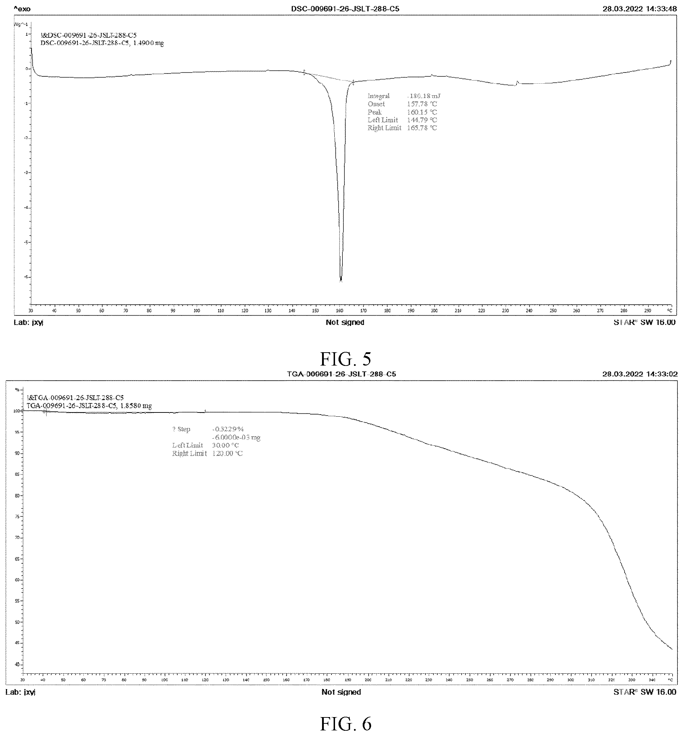

Preferably, the crystal form B has a DSC profile substantially as shown in FIG. 5.

Preferably, the crystal form B has a TGA profile substantially as shown in FIG. 6.

The present disclosure provides a crystal form C of compound I, which has an X-ray powder diffraction pattern comprising peaks at diffraction angles (2θ) of 10.90±0.2°, 5.80±0.2°, 19.28±0.2°, and 14.49±0.2°.

According to the present disclosure, preferably, the crystal form C further comprises peaks at diffraction angles (2θ) of 15.37±0.2°, 12.95±0.2°, 19.77±0.2°, 3.11±0.2°, and 24.46±0.2°.

According to the present disclosure, more preferably, the crystal form C further comprises peaks at diffraction angles (2θ) of 16.38±0.2°, 25.22±0.2°, 19.03±0.2°, 20.82±0.2°, 18.00±0.2°, and 18.15±0.2°.

According to the present disclosure, preferably, the crystal form C has an X-ray powder diffraction pattern comprising diffraction angles (2θ) shown in Table 3, wherein the 2θ angles have a margin of error of ±0.20°:

**Table 3**

| 2θ (°) | Intensity% | 2θ (°) | Intensity% |
|---|---|---|---|
| 3.11 | 16.8 | 19.77 | 20.1 |
| 3.60 | 11.0 | 20.02 | 8.5 |
| 5.40 | 11.2 | 20.45 | 7.5 |
| 5.80 | 29.1 | 20.82 | 13.6 |
| 6.91 | 6.1 | 21.08 | 9.4 |
| 8.07 | 7.9 | 21.42 | 8.2 |
| 8.80 | 7.6 | 21.99 | 10.1 |
| 9.00 | 6.0 | 22.62 | 9.0 |
| 9.29 | 3.4 | 22.77 | 9.1 |
| 9.88 | 9.1 | 23.20 | 12.0 |
| 10.90 | 100.0 | 23.93 | 6.8 |
| 12.46 | 4.4 | 24.21 | 12.0 |
| 12.95 | 23.8 | 24.46 | 16.6 |
| 13.57 | 10.8 | 24.87 | 3.6 |
| 13.79 | 3.4 | 25.22 | 14.3 |
| 14.18 | 7.2 | 25.83 | 6.4 |
| 14.29 | 9.4 | 26.45 | 3.2 |
| 14.49 | 26.1 | 26.67 | 5.2 |
| 14.68 | 6.7 | 26.96 | 7.4 |
| 14.94 | 5.0 | 28.15 | 5.2 |
| 15.37 | 25.1 | 28.63 | 3.0 |
| 15.95 | 8.2 | 28.81 | 5.6 |
| 16.38 | 15.7 | 30.11 | 2.1 |
| 16.63 | 6.5 | 30.34 | 3.4 |
| 18.00 | 13.2 | 31.34 | 1.9 |
| 18.15 | 12.6 | 32.04 | 2.0 |
| 18.69 | 8.3 | 34.83 | 2.9 |
| 19.03 | 13.9 | 35.25 | 2.6 |
| 19.28 | 27.5 | 36.37 | 2.0 |
| 19.57 | 8.7 | | |

Preferably, the crystal form C has X-ray powder diffraction intensities shown in Table 3.

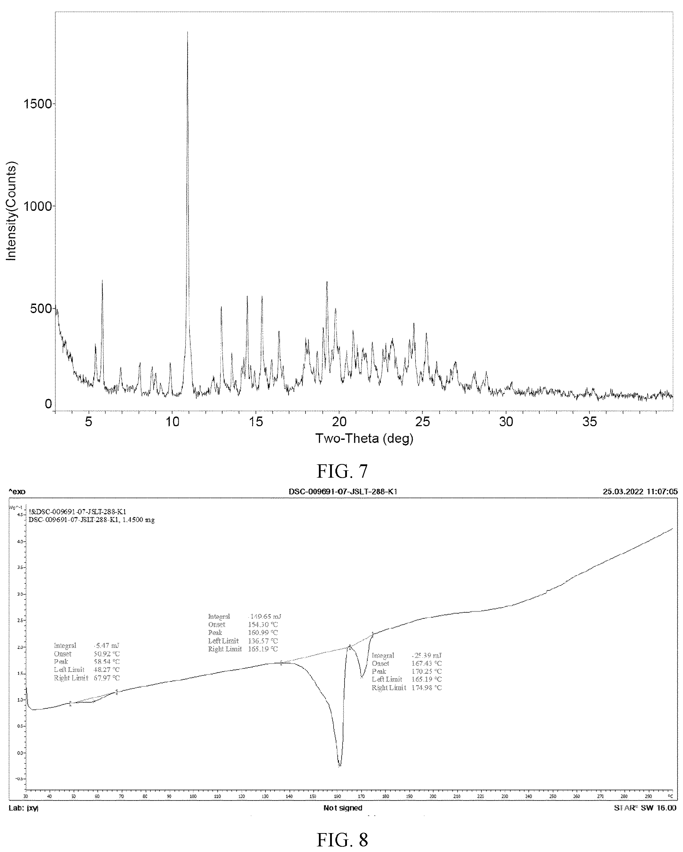

Preferably, the crystal form C has an X-ray powder diffraction pattern substantially as shown in FIG. 7.

Preferably, the crystal form C exhibits endothermic peaks in a DSC analysis when heated to peak temperatures of about 160.99 °C and about 170.25 °C.

Preferably, the crystal form C has a DSC profile substantially as shown in FIG. 8.

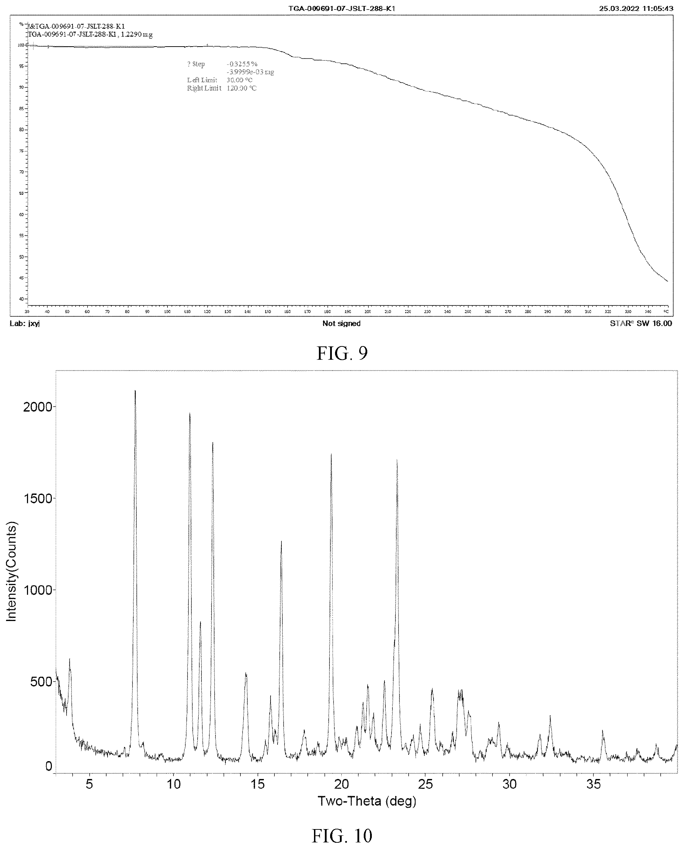

Preferably, the crystal form C has a TGA profile substantially as shown in FIG. 9.

The present disclosure provides a hydrate crystal form D of compound I, which has an X-ray powder diffraction pattern comprising peaks at diffraction angles (2θ) of 7.71±0.2°, 10.96±0.2°, 12.34±0.2°, and 19.38±0.2°.

According to the present disclosure, preferably, the crystal form D further comprises peaks at diffraction angles (2θ) of 23.30±0.2°, 16.42±0.2°, 11.60±0.2°, 14.30±0.2°, and 3.81±0.2°.

According to the present disclosure, more preferably, the crystal form D further comprises peaks at diffraction angles (2θ) of 21.54±0.2°, 22.55±0.2°, 25.40±0.2°, 26.96±0.2°, 15.78±0.2°, and 21.29±0.2°.

According to the present disclosure, preferably, the crystal form D has an X-ray powder diffraction pattern comprising diffraction angles (2θ) shown in Table 4, wherein the 2θ angles have a margin of error of ±0.20°:

**Table 4**

| 2θ (°) | Intensity% | 2θ (°) | Intensity% |
|---|---|---|---|
| 3.81 | 23.9 | 21.91 | 9.7 |
| 7.08 | 2.1 | 22.55 | 18.1 |
| 7.71 | 100.0 | 23.30 | 79.6 |
| 8.21 | 4.1 | 24.27 | 4.8 |
| 9.32 | 1.8 | 24.68 | 7.7 |
| 10.96 | 94.0 | 25.40 | 17.4 |
| 11.60 | 36 | 25.91 | 2.5 |
| 12.34 | 85.6 | 26.96 | 17.4 |
| 14.30 | 24.1 | 27.54 | 11.4 |
| 15.46 | 3.4 | 28.24 | 2.0 |
| 15.78 | 17.2 | 28.73 | 4.9 |
| 16.03 | 7.8 | 28.96 | 5.3 |
| 16.42 | 58.1 | 29.35 | 9.3 |
| 17.80 | 6.8 | 29.88 | 3.9 |
| 18.58 | 3.3 | 31.81 | 5.9 |
| 19.38 | 81.6 | 32.41 | 11.1 |
| 19.85 | 4.7 | 35.53 | 8.1 |
| 20.29 | 3.7 | 36.95 | 2.1 |
| 20.90 | 6.3 | 37.59 | 3.0 |
| 21.29 | 13.6 | 38.72 | 4.7 |
| 21.54 | 18.4 | | |

Preferably, the crystal form D has X-ray powder diffraction intensities shown in Table 4.

Preferably, the crystal form D has an X-ray powder diffraction pattern substantially as shown in FIG. 10.

Preferably, the crystal form D exhibits endothermic peaks in a DSC analysis when heated to peak temperatures of about 62.09 °C, about 79.77 °C, and about 173.12 °C.

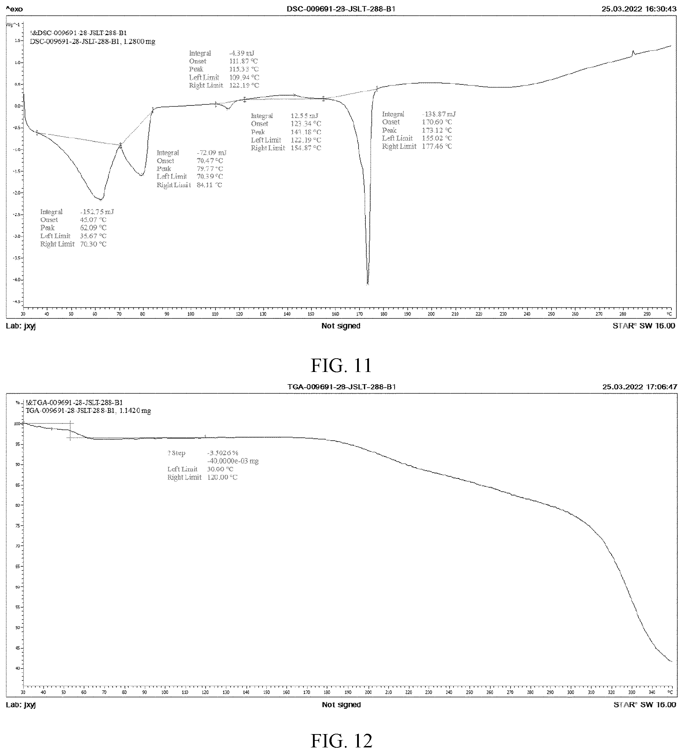

Preferably, the crystal form D has a DSC profile substantially as shown in FIG. 11.

Preferably, the crystal form D has a TGA profile substantially as shown in FIG. 12.

The present disclosure provides a trichloromethane solvate crystal form E of compound I, which has an X-ray powder diffraction pattern comprising peaks at diffraction angles (2θ) of 18.84±0.2°, 7.26±0.2°, 22.92±0.2°, and 9.35±0.2°.

According to the present disclosure, preferably, the crystal form E further comprises peaks at diffraction angles (2θ) of 22.05±0.2°, 18.36±0.2°, 6.21±0.2°, 3.60±0.2°, and 16.98±0.2°.

According to the present disclosure, more preferably, the crystal form E further comprises peaks at diffraction angles (2θ) of 12.50±0.2°, 34.99±0.2°, 16.11±0.2°, 20.51±0.2°, 19.57±0.2°, and 17.57±0.2°.

According to the present disclosure, preferably, the crystal form E has an X-ray powder diffraction pattern comprising diffraction angles (2θ) shown in Table 5, wherein the 2θ angles have a margin of error of ±0.20°:

**Table 5**

| 2θ (°) | Intensity% | 2θ (°) | Intensity% |
|---|---|---|---|
| 3.60 | 21.9 | 20.51 | 13.9 |
| 6.21 | 34.6 | 20.92 | 3.3 |
| 7.26 | 78.8 | 22.05 | 37.7 |
| 9.35 | 55.3 | 22.92 | 59.1 |
| 10.12 | 1.7 | 23.53 | 4.8 |
| 11.04 | 2.0 | 23.95 | 1.9 |
| 12.50 | 16.4 | 24.36 | 4.4 |
| 14.18 | 4.4 | 24.81 | 2.1 |
| 15.11 | 1.6 | 25.23 | 2.1 |
| 15.66 | 3.7 | 26.12 | 1.8 |
| 16.11 | 14.2 | 26.94 | 1.7 |
| 16.61 | 6.3 | 28.48 | 5.4 |
| 16.98 | 20.7 | 29.09 | 2.7 |
| 17.57 | 9.6 | 29.37 | 2.0 |
| 18.01 | 1.9 | 29.64 | 1.7 |
| 18.36 | 35.2 | 31.69 | 4.8 |
| 18.84 | 100.0 | 32.27 | 2.0 |
| 19.57 | 11.4 | 33.41 | 7.6 |
| 19.90 | 7.1 | 34.99 | 15.3 |

Preferably, the crystal form E has X-ray powder diffraction intensities shown in Table 5.

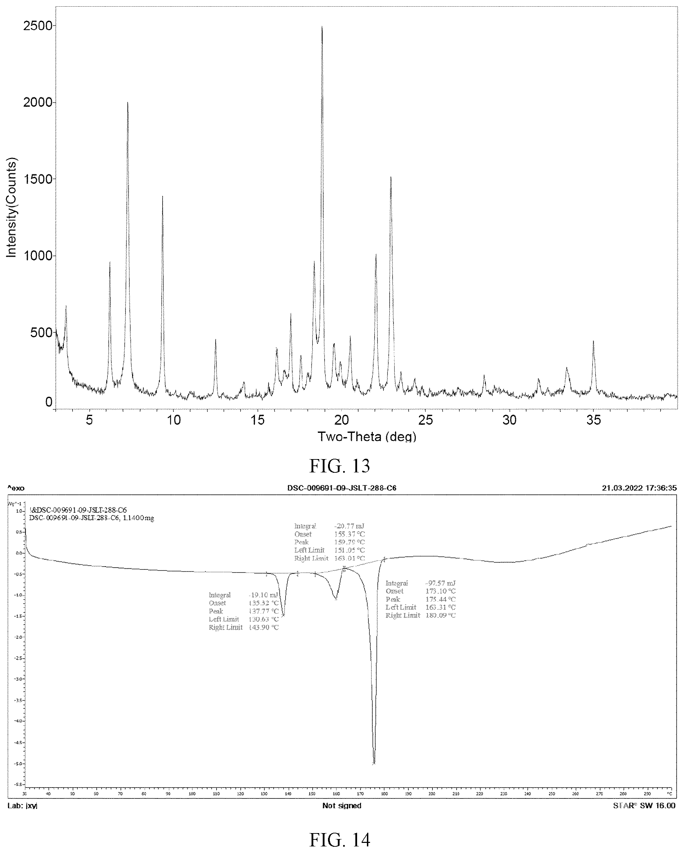

Preferably, the crystal form E has an X-ray powder diffraction pattern substantially as shown in FIG. 13.

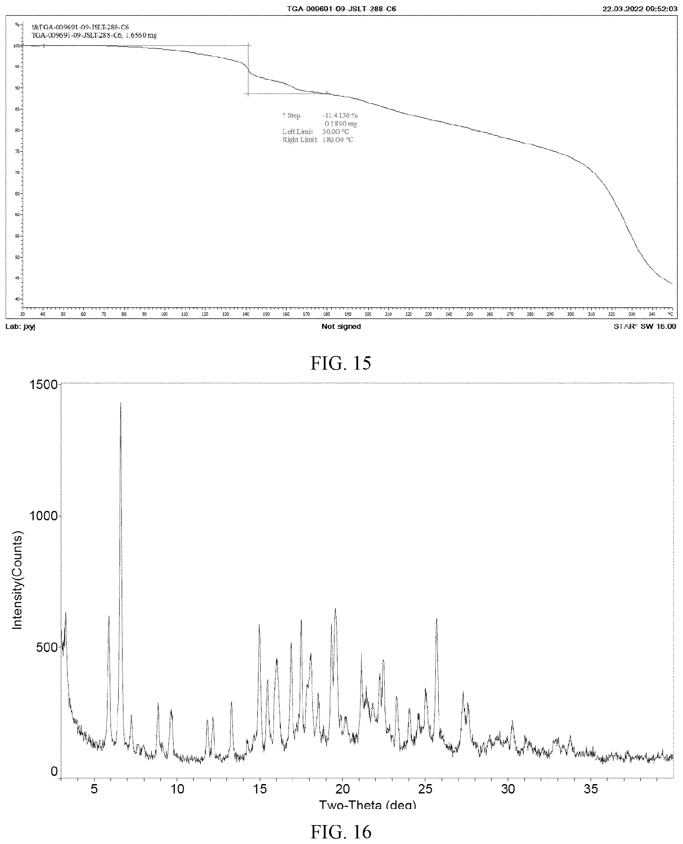

Preferably, the crystal form E exhibits endothermic peaks in a DSC analysis when heated to peak temperatures of about 137.77 °C, about 159.79 °C, and about 175.44 °C. Preferably, the crystal form E has a DSC profile substantially as shown in FIG. 14. Preferably, the crystal form E has a TGA profile substantially as shown in FIG. 15.

The present disclosure provides a N-methylpyrrolidone solvate crystal form F of compound I, which has an X-ray powder diffraction pattern comprising peaks at diffraction angles (2θ) of 6.60±0.2°, 19.57±0.2°, 5.89±0.2°, and 14.96±0.2°. According to the present disclosure, preferably, the crystal form F further comprises peaks at diffraction angles (2θ) of 25.69±0.2°, 3.27±0.2°, 17.49±0.2°, 19.32±0.2°, and 16.89±0.2°.

According to the present disclosure, more preferably, the crystal form F further comprises peaks at diffraction angles (2θ) of 16.01±0.2°, 18.07±0.2°, 21.13±0.2°, 22.46±0.2°, 15.48±0.2°, and 27.29±0.2°.

According to the present disclosure, preferably, the crystal form F has an X-ray powder diffraction pattern comprising diffraction angles (2θ) shown in Table 6, wherein the 2θ angles have a margin of error of ±0.20°:

**Table 6**

| 2θ (°) | Intensity% | 2θ (°) | Intensity% |
|---|---|---|---|
| 3.27 | 34.5 | 21.83 | 8.5 |
| 5.89 | 37.2 | 22.24 | 15.3 |
| 6.60 | 100.0 | 22.46 | 19.8 |
| 7.24 | 9.7 | 23.24 | 13.0 |
| 8.86 | 15.5 | 24.03 | 9.2 |
| 9.10 | 3.9 | 24.61 | 8.5 |
| 9.66 | 13.8 | 25.01 | 14.4 |
| 11.82 | 11.8 | 25.69 | 35.5 |
| 12.15 | 12.6 | 27.29 | 17.6 |
| 13.28 | 16.4 | 27.54 | 14.4 |
| 14.64 | 5.3 | 28.90 | 3.0 |
| 14.96 | 36.2 | 29.35 | 3.7 |
| 15.48 | 19.6 | 29.35 | 3.7 |
| 16.01 | 25.8 | 29.97 | 3.6 |
| 16.89 | 28.0 | 30.25 | 7.8 |
| 17.49 | 34.3 | 31 | 4.9 |
| 18.07 | 24.4 | 31.30 | 3.6 |
| 18.52 | 11.8 | 32.82 | 4.0 |
| 19.32 | 32.8 | 33.05 | 5.2 |
| 19.57 | 38.1 | 33.37 | 3.0 |
| 20.18 | 4.5 | 33.76 | 5.2 |
| 21.13 | 23.3 | 37.25 | 2.4 |
| 21.42 | 12.5 | 39.32 | 2.7 |

Preferably, the crystal form F has X-ray powder diffraction intensities shown in Table 6.

Preferably, the crystal form F has an X-ray powder diffraction pattern substantially as shown in FIG. 16.

Preferably, the crystal form F exhibits endothermic peaks in a DSC analysis when heated to peak temperatures of about 72.65 °C, about 95.65 °C, about 127.81 °C, and about 159.14 °C.

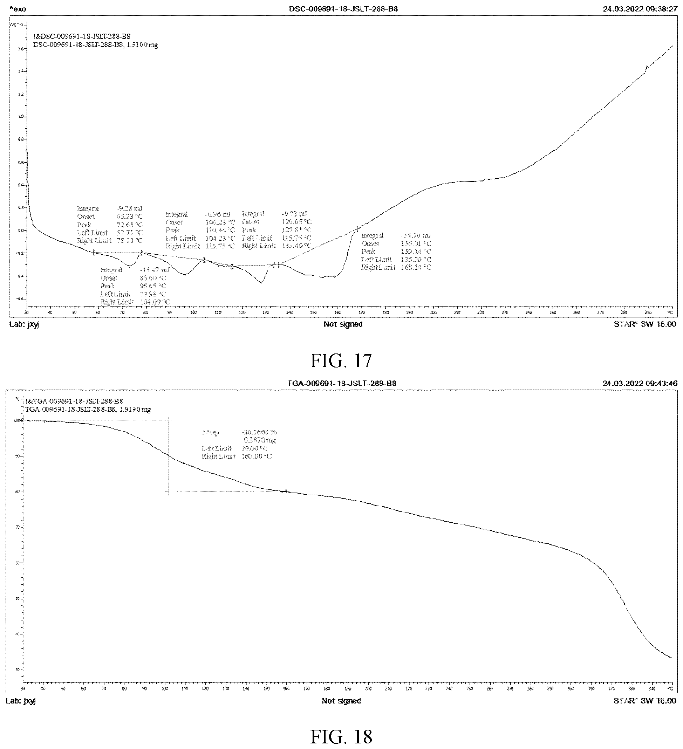

Preferably, the crystal form F has a DSC profile substantially as shown in FIG. 17.

Preferably, the crystal form F has a TGA profile substantially as shown in FIG. 18.

The present disclosure provides a N-methylpyrrolidone solvate crystal form G of compound I, which has an X-ray powder diffraction pattern comprising peaks at diffraction angles (2θ) of 3.50±0.2°, 6.97±0.2°, 13.91±0.2°, and 22.19±0.2°.

According to the present disclosure, preferably, the crystal form G further comprises peaks at diffraction angles (2θ) of 31.61±0.2°, 18.11±0.2°, 20.55±0.2°, 18.97±0.2°, and 15.76±0.2°.

According to the present disclosure, more preferably, the crystal form G further comprises peaks at diffraction angles (2θ) of 28.52±0.2°, 35.16±0.2°, 20.86±0.2°, 16.36±0.2°, 26.02±0.2°, and 17.18±0.2°.

According to the present disclosure, preferably, the crystal form G has an X-ray powder diffraction pattern comprising diffraction angles (2θ) shown in Table 7, wherein the 2θ angles have a margin of error of ±0.20°:

**Table 7**

| 2θ(°) | Intensity% | 2θ(°) | Intensity% |
|---|---|---|---|
| 3.50 | 100.0 | 20.55 | 14.0 |
| 6.97 | 98.6 | 20.86 | 9.0 |
| 10.16 | 6.1 | 22.19 | 28.8 |
| 11.06 | 5.1 | 23.43 | 2.9 |
| 11.49 | 2.8 | 23.88 | 6.0 |
| 13.91 | 35.2 | 24.27 | 3.5 |
| 14.41 | 5.2 | 26.02 | 8.2 |
| 14.92 | 2.3 | 27.09 | 6.1 |
| 15.76 | 11.9 | 27.48 | 4.0 |
| 16.36 | 8.7 | 27.99 | 5.3 |
| 16.91 | 3.9 | 28.52 | 11.0 |
| 17.18 | 6.5 | 30.27 | 5.1 |
| 17.80 | 4.8 | 31.61 | 18.4 |
| 18.11 | 17.3 | 33.11 | 3.4 |
| 18.97 | 13.9 | 35.16 | 9.7 |
| 19.71 | 3.9 | 38.80 | 2.5 |

Preferably, the crystal form G has X-ray powder diffraction intensities shown in Table 7.

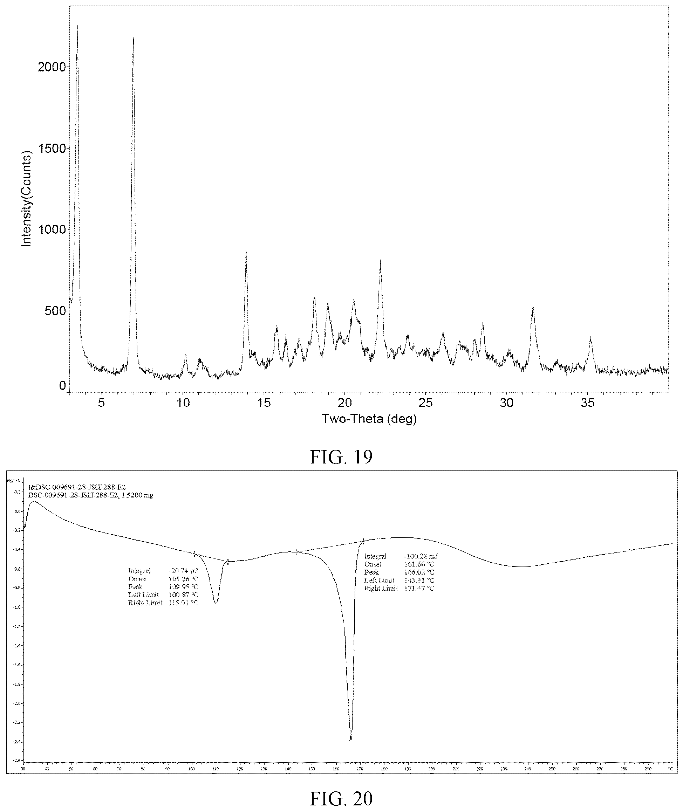

Preferably, the crystal form G has an X-ray powder diffraction pattern substantially as shown in FIG. 19.

Preferably, the crystal form G exhibits endothermic peaks in a DSC analysis when heated to peak temperatures of about 109.95 °C and about 166.02 °C.

Preferably, the crystal form G has a DSC profile substantially as shown in FIG. 20.

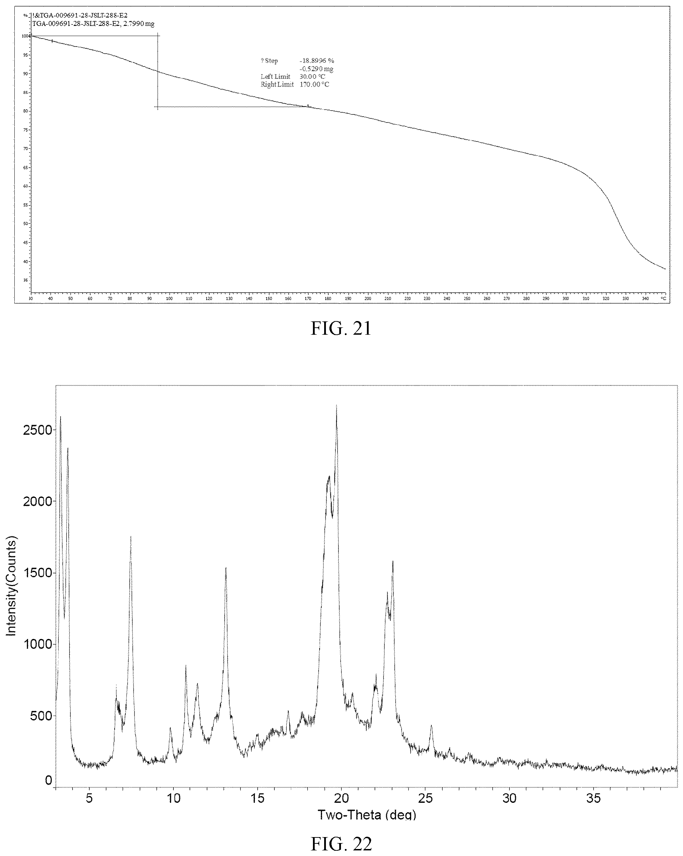

Preferably, the crystal form G has a TGA profile substantially as shown in FIG. 21.

The present disclosure provides a tetrahydrofuran solvate crystal form H of compound I, which has an X-ray powder diffraction pattern comprising peaks at diffraction angles (2θ) of 3.29±0.2°, 3.71±0.2°, 19.71±0.2°, and 19.24±0.2°.

According to the present disclosure, preferably, the crystal form H further comprises peaks at diffraction angles (2θ) of 7.46±0.2°, 13.12±0.2°, 23.08±0.2°, 22.73±0.2°, and 6.60±0.2°.

According to the present disclosure, more preferably, the crystal form H further comprises peaks at diffraction angles (2θ) of 10.73±0.2°, 11.43±0.2°, 22.05±0.2°, 9.82±0.2°, 25.34±0.2°, and 16.83±0.2°.

According to the present disclosure, preferably, the crystal form H has an X-ray powder diffraction pattern comprising diffraction angles (2θ) shown in Table 8, wherein the 2θ angles have a margin of error of ±0.20°:

**Table 8**

| 2θ (°) | Intensity% | 2θ (°) | Intensity% |
|---|---|---|---|
| 3.29 | 100.0 | 17.67 | 4.6 |
| 3.71 | 92.5 | 19.24 | 67.6 |
| 6.60 | 22.9 | 19.71 | 80.8 |
| 7.46 | 63.0 | 22.05 | 11.8 |
| 9.82 | 9.2 | 22.73 | 40.5 |
| 10.73 | 22.4 | 23.08 | 48.1 |
| 11.43 | 18.1 | 25.34 | 8.5 |
| 13.12 | 52.5 | 26.43 | 3.1 |
| 15.03 | 4.2 | 27.66 | 2.7 |
| 15.88 | 3.0 | 29.39 | 2.0 |
| 16.49 | 3.4 | 31.10 | 2.2 |
| 16.83 | 7.2 | 35.51 | 1.7 |

Preferably, the crystal form H has X-ray powder diffraction intensities shown in Table 8.

Preferably, the crystal form H has an X-ray powder diffraction pattern substantially as shown in FIG. 22.

Preferably, the crystal form H exhibits endothermic peaks in a DSC analysis when heated to peak temperatures of about 82.80 °C and about 174.84 °C.

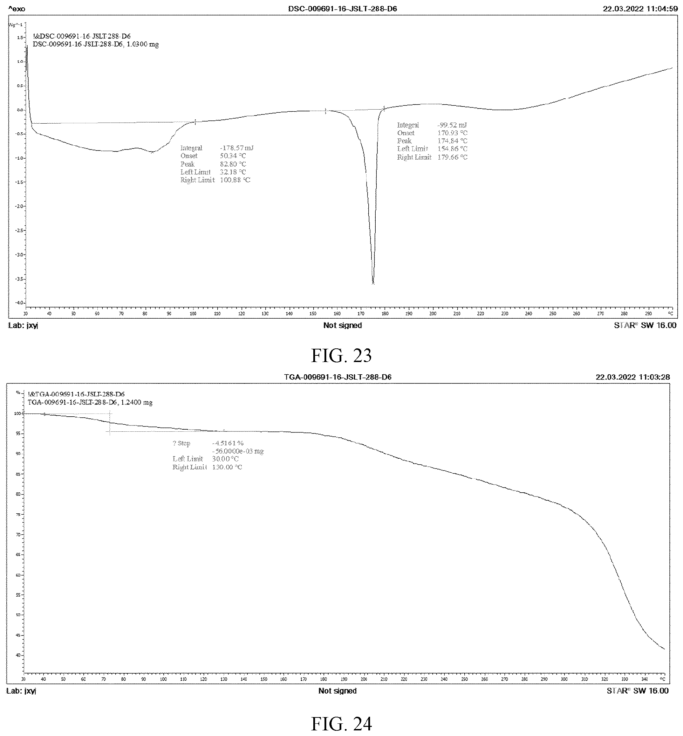

Preferably, the crystal form H has a DSC profile substantially as shown in FIG. 23.

Preferably, the crystal form H has a TGA profile substantially as shown in FIG. 24.

The present disclosure provides an ethanol solvate crystal form I of compound I, which has an X-ray powder diffraction pattern comprising peaks at diffraction angles (2θ) of 18.97±0.2°, 10.71±0.2°, 16.19±0.2°, and 22.67±0.2°.

According to the present disclosure, preferably, the crystal form I further comprises peaks at diffraction angles (2θ) of 12.79±0.2°, 15.13±0.2°, 17.99±0.2°, 22.52±0.2°, and 23.94±0.2°.

According to the present disclosure, more preferably, the crystal form I further comprises peaks at diffraction angles (2θ) of 16.53±0.2°, 24.99±0.2°, 21.85±0.2°, 19.28±0.2°, 26.69±0.2°, and 24.38±0.2°.

According to the present disclosure, preferably, the crystal form I has an X-ray powder diffraction pattern comprising diffraction angles (2θ) shown in Table 9, wherein the 2θ angles have a margin of error of ±0.20°:

**Table 9**

| 2θ (°) | Intensity% | 2θ (°) | Intensity% |
|---|---|---|---|
| 5.30 | 6.6 | 22.67 | 31.8 |
| 6.36 | 1.4 | 22.87 | 13.3 |
| 6.94 | 12.2 | 23.16 | 5.0 |
| 8.77 | 4.6 | 23.46 | 5.5 |
| 10.71 | 92.6 | 23.94 | 16.6 |
| 11.25 | 3.3 | 24.38 | 13.6 |
| 11.77 | 1.8 | 24.99 | 15.4 |
| 12.38 | 1.6 | 25.71 | 8.7 |
| 12.79 | 29.5 | 25.94 | 4.5 |
| 13.24 | 8.7 | 26.31 | 4.6 |
| 13.92 | 5.5 | 26.69 | 14.1 |
| 14.20 | 3.9 | 27.16 | 4.0 |
| 14.56 | 10.8 | 27.83 | 6.9 |
| 15.13 | 25.0 | 28.44 | 4.8 |
| 15.40 | 4.5 | 28.75 | 2.6 |
| 16.19 | 35.0 | 29.13 | 2.2 |
| 16.53 | 15.4 | 29.92 | 3.2 |
| 17.08 | 2.1 | 30.34 | 2.6 |
| 17.35 | 2.5 | 31.12 | 1.3 |
| 17.61 | 6.7 | 31.38 | 1.9 |
| 17.99 | 22.5 | 31.95 | 3.9 |
| 18.97 | 100.0 | 32.29 | 1.7 |
| 19.28 | 14.1 | 32.88 | 2.5 |
| 19.87 | 12.6 | 33.36 | 1.6 |
| 20.20 | 13.1 | 33.74 | 1.2 |
| 20.57 | 2.9 | 34.98 | 1.8 |
| 21.07 | 12.2 | 35.92 | 1.9 |
| 21.27 | 9.4 | 36.42 | 1.9 |
| 21.70 | 9.5 | 37.31 | 1.4 |
| 21.85 | 14.6 | 38.45 | 1.3 |
| 22.52 | 19.7 | 39.21 | 1.9 |

Preferably, the crystal form I has X-ray powder diffraction intensities shown in Table 9.

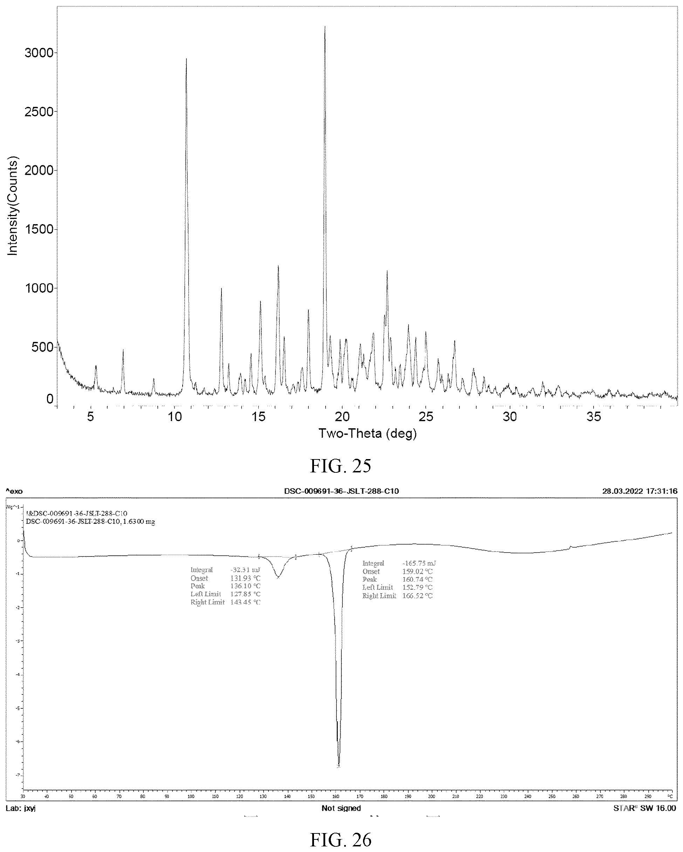

Preferably, the crystal form I has an X-ray powder diffraction pattern substantially as shown in FIG. 25.

Preferably, the crystal form I exhibits endothermic peaks in a DSC analysis when heated to peak temperatures of about 136.10 °C and about 160.74 °C.

Preferably, the crystal form I has a DSC profile substantially as shown in FIG. 26.

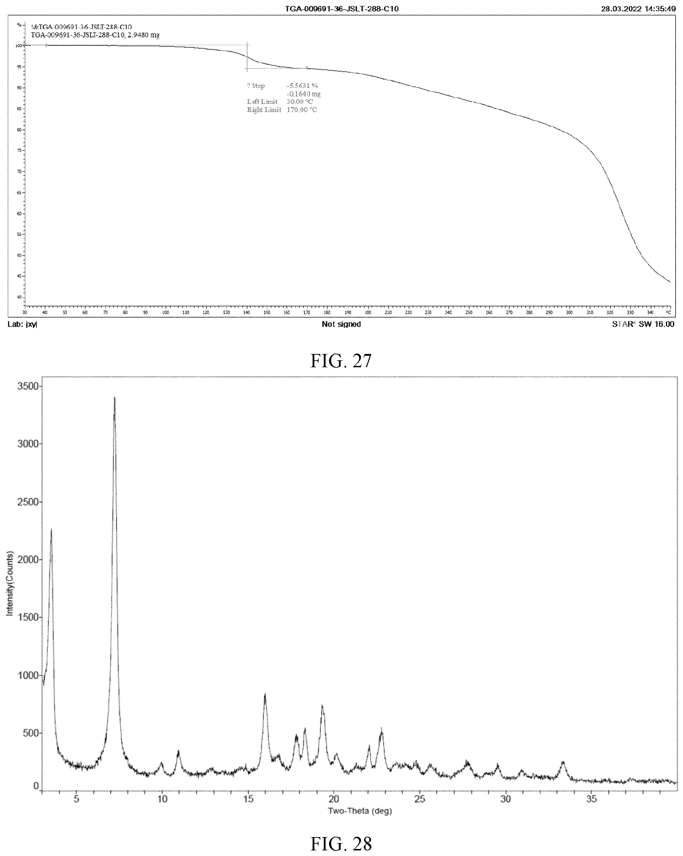

Preferably, the crystal form I has a TGA profile substantially as shown in FIG. 27.

The present disclosure provides a metastable crystal form J of compound I, which has an X-ray powder diffraction pattern comprising peaks at diffraction angles (2θ) of 7.22±0.2°, 3.54±0.2°, 15.99±0.2°, and 19.34±0.2°.

According to the present disclosure, preferably, the crystal form J further comprises peaks at diffraction angles (2θ) of 22.77±0.2°, 18.33±0.2°, 17.80±0.2°, 10.93±0.2°, and 22.07±0.2°.

According to the present disclosure, more preferably, the crystal form J further comprises peaks at diffraction angles (2θ) of 33.38±0.2°, 27.77±0.2°, 29.53±0.2°, 9.95±0.2°, 20.16±0.2°, and 25.56±0.2°.

According to the present disclosure, preferably, the crystal form J has an X-ray powder diffraction pattern comprising diffraction angles (2θ) shown in Table 10, wherein the 2θ angles have a margin of error of ±0.20°:

**Table 10**

| 2θ (°) | Intensity% | 2θ (°) | Intensity% |
|---|---|---|---|
| 3.54 | 63.8 | 21.30 | 2.1 |
| 7.22 | 100.0 | 22.07 | 5.9 |
| 9.95 | 3.6 | 22.77 | 11.3 |
| 10.93 | 6.7 | 23.63 | 2.0 |
| 12.95 | 1.9 | 24.20 | 2.1 |
| 14.61 | 1.7 | 24.79 | 2.8 |
| 14.86 | 2.4 | 25.56 | 2.9 |
| 15.99 | 20.5 | 27.77 | 4.5 |
| 16.81 | 2.6 | 28.85 | 1.3 |
| 17.80 | 9.0 | 29.53 | 4.0 |
| 18.33 | 10.3 | 30.91 | 2.2 |
| 19.34 | 15.6 | 33.38 | 5 |
| 20.16 | 3.6 | 39.45 | 1.0 |

Preferably, the crystal form J has X-ray powder diffraction intensities shown in Table 10.

Preferably, the crystal form J has an X-ray powder diffraction pattern substantially as shown in FIG. 28.

The present disclosure provides a metastable crystal form K of compound I, which has an X-ray powder diffraction pattern comprising peaks at diffraction angles (2θ) of 6.13±0.2°, 7.19±0.2°, 3.56±0.2°, and 22.63±0.2°.

According to the present disclosure, preferably, the crystal form K further comprises peaks at diffraction angles (2θ) of 18.56±0.2°, 12.34±0.2°, 16.83±0.2°, 9.31±0.2°, and 26.20±0.2°.

According to the present disclosure, more preferably, the crystal form K further comprises peaks at diffraction angles (2θ) of 19.63±0.2°, 20.26±0.2°, 23.72±0.2°, 17.40±0.2°, 34.48±0.2°, and 24.21±0.2°.

According to the present disclosure, preferably, the crystal form K has an X-ray powder diffraction pattern comprising diffraction angles (2θ) shown in Table 11, wherein the 2θ angles have a margin of error of ±0.20°:

**Table 11**

| 2θ (°) | Intensity% | 2θ (°) | Intensity% |
|---|---|---|---|
| 3.56 | 35.1 | 21.68 | 6.1 |
| 6.13 | 100.0 | 22.00 | 3.1 |
| 7.19 | 39.3 | 22.63 | 31.4 |
| 9.31 | 12.1 | 23.20 | 2.5 |
| 11.51 | 2.2 | 23.72 | 8.4 |
| 12.34 | 17.9 | 24.21 | 7.8 |
| 14.06 | 5.0 | 24.99 | 3.0 |
| 14.53 | 4.9 | 26.20 | 10.1 |
| 15.62 | 5.6 | 28.48 | 5.9 |
| 16.83 | 17.6 | 29.53 | 3.4 |
| 17.40 | 8.3 | 30.11 | 2.7 |
| 17.79 | 2.6 | 31.26 | 7.4 |
| 18.56 | 21.7 | 31.94 | 2.0 |
| 19.12 | 3.8 | 34.48 | 8.1 |
| 19.63 | 9.9 | 35.53 | 2.3 |
| 20.26 | 8.5 | 38.75 | 1.9 |
| 20.82 | 7.7 | | |

Preferably, the crystal form K has X-ray powder diffraction intensities shown in Table 11.

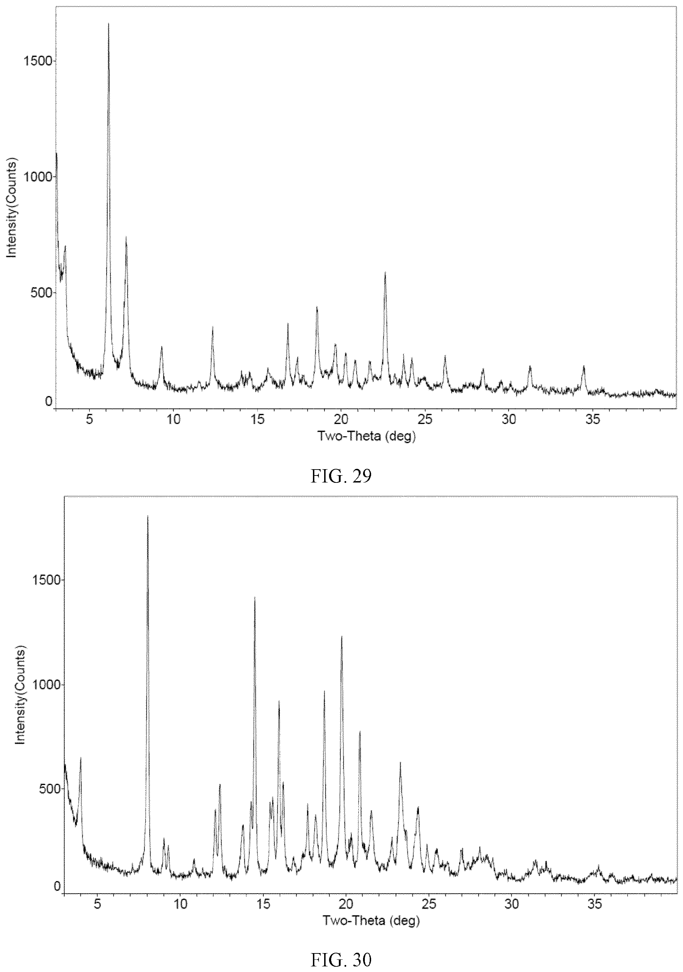

Preferably, the crystal form K has an X-ray powder diffraction pattern substantially as shown in FIG. 29.

The present disclosure provides a metastable crystal form L of compound I, which has an X-ray powder diffraction pattern comprising peaks at diffraction angles (2θ) of 8.04±0.2°, 14.49±0.2°, 19.75±0.2°, and 15.95±0.2°.

According to the present disclosure, preferably, the crystal form L further comprises peaks at diffraction angles (2θ) of 18.70±0.2°, 20.85±0.2°, 4.00±0.2°, 23.27±0.2°, and 12.40±0.2°.

According to the present disclosure, more preferably, the crystal form L further comprises peaks at diffraction angles (2θ) of 16.22±0.2°, 15.58±0.2°, 14.27±0.2°, 12.13±0.2°, 15.43±0.2°, and 17.69±0.2°.

According to the present disclosure, preferably, the crystal form L has an X-ray powder diffraction pattern comprising diffraction angles (2θ) shown in Table 12, wherein the 2θ angles have a margin of error of ±0.20°:

**Table 12**

| 2θ (°) | Intensity% | 2θ (°) | Intensity% |
|---|---|---|---|
| 4.00 | 29.7 | 21.54 | 16.0 |
| 8.04 | 100.0 | 22.78 | 6.9 |
| 9.02 | 9.9 | 23.27 | 29.4 |
| 9.29 | 8.1 | 23.63 | 9.6 |
| 10.84 | 4.8 | 24.36 | 17.2 |
| 12.13 | 18.7 | 24.89 | 7.2 |
| 12.40 | 25.8 | 25.46 | 6.2 |
| 13.79 | 13.3 | 26.10 | 3.2 |
| 14.27 | 20.0 | 26.96 | 5.9 |
| 14.49 | 77.0 | 28.07 | 7.2 |
| 15.43 | 18.5 | 28.51 | 5.3 |
| 15.58 | 20.7 | 28.86 | 2.5 |
| 15.95 | 47.5 | 29.69 | 2.8 |
| 16.22 | 24.6 | 31.43 | 5.4 |
| 16.84 | 3.9 | 31.82 | 2.7 |
| 17.39 | 3.9 | 32.08 | 3.9 |
| 17.69 | 17.2 | 32.35 | 2.4 |
| 18.17 | 14.6 | 35.24 | 4.4 |
| 18.70 | 47.5 | 36.07 | 2.4 |
| 19.75 | 63.9 | 37.30 | 1.9 |
| 20.31 | 8.5 | 38.44 | 2.3 |
| 20.85 | 37.2 | | |

Preferably, the crystal form L has X-ray powder diffraction intensities shown in Table 12.

Preferably, the crystal form L has an X-ray powder diffraction pattern substantially as shown in FIG. 30.

The present disclosure provides a metastable crystal form M of compound I, which has an X-ray powder diffraction pattern comprising peaks at diffraction angles (2θ) of 19.96±0.2°, 12.72±0.2°, 7.92±0.2°, and 11.12±0.2°.

According to the present disclosure, preferably, the crystal form M further comprises peaks at diffraction angles (2θ) of 16.54±0.2°, 3.95±0.2°, 21.66±0.2°, 24.01±0.2°, and 22.69±0.2°.

According to the present disclosure, more preferably, the crystal form M further comprises peaks at diffraction angles (2θ) of 14.45±0.2°, 11.92±0.2°, 15.93±0.2°, 20.98±0.2°, 23.59±0.2°, and 6.61±0.2°.

According to the present disclosure, preferably, the crystal form M has an X-ray powder diffraction pattern comprising diffraction angles (2θ) shown in Table 13, wherein the 2θ angles have a margin of error of ±0.20°:

**Table 13**

| 2θ (°) | Intensity% | 2θ (°) | Intensity% |
|---|---|---|---|
| 3.95 | 55.2 | 22.69 | 27.5 |
| 6.61 | 7.2 | 23.10 | 2.8 |
| 7.92 | 67.6 | 23.59 | 8.3 |
| 9.49 | 3.7 | 24.01 | 35.2 |
| 9.98 | 2.2 | 24.69 | 3.9 |
| 11.12 | 65.9 | 26.12 | 2.9 |
| 11.92 | 20.5 | 26.49 | 3.7 |
| 12.72 | 84.2 | 26.72 | 6.1 |
| 13.84 | 2.5 | 27.33 | 2.8 |
| 14.45 | 26.4 | 27.79 | 6.1 |
| 14.80 | 2.5 | 29.12 | 3.7 |
| 15.93 | 10.3 | 30.22 | 5.2 |
| 16.54 | 56.6 | 32.74 | 3.9 |
| 17.77 | 2.8 | 32.96 | 4.5 |
| 18.21 | 7.1 | 33.17 | 4.0 |
| 19.96 | 100.0 | 36.42 | 2.1 |
| 20.98 | 8.8 | 36.78 | 2.8 |
| 21.66 | 42.7 | 39.08 | 1.7 |

Preferably, the crystal form M has X-ray powder diffraction intensities shown in Table 13.

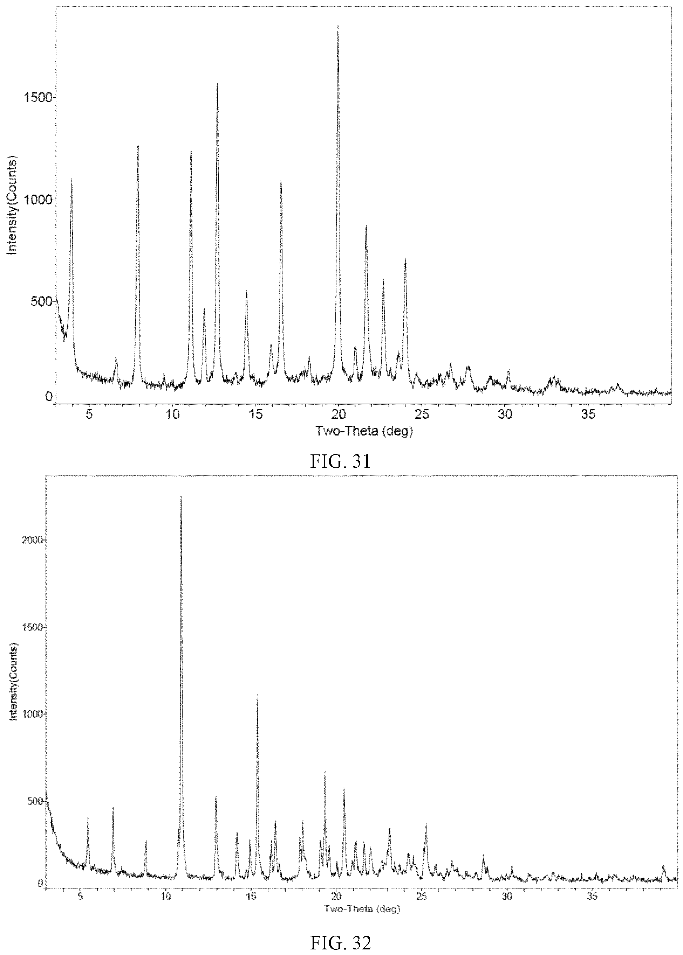

Preferably, the crystal form M has an X-ray powder diffraction pattern substantially as shown in FIG. 31.

The present disclosure provides a metastable crystal form N of compound I, which has an X-ray powder diffraction pattern comprising peaks at diffraction angles (2θ) of 10.92±0.2°, 15.37±0.2°, 19.34±0.2°, and 20.46±0.2°.

According to the present disclosure, preferably, the crystal form N further comprises peaks at diffraction angles (2θ) of 12.95±0.2°, 6.93±0.2°, 18.04±0.2°, 16.43±0.2°, and 25.26±0.2°.

According to the present disclosure, more preferably, the crystal form N further comprises peaks at diffraction angles (2θ) of 5.43±0.2°, 10.75±0.2°, 14.20±0.2°, 23.12±0.2°, 17.87±0.2°, and 14.94±0.2°.

According to the present disclosure, preferably, the crystal form N has an X-ray powder diffraction pattern comprising diffraction angles (2θ) shown in Table 14, wherein the 2θ angles have a margin of error of ±0.20°:

**Table 14**

| 2θ (°) | Intensity% | 2θ (°) | Intensity% |
|---|---|---|---|
| 5.43 | 13.3 | 23.70 | 2.4 |
| 6.93 | 17.2 | 23.93 | 1.2 |
| 7.42 | 2.0 | 24.23 | 5.6 |
| 8.85 | 9.7 | 24.50 | 5.3 |
| 10.75 | 12.7 | 25.26 | 14.4 |
| 10.92 | 100.0 | 25.83 | 3.7 |
| 12.95 | 21.6 | 26.10 | 1.5 |
| 14.20 | 12.0 | 26.47 | 2.7 |
| 14.71 | 1.4 | 26.78 | 4.6 |
| 14.94 | 10.1 | 27.09 | 2.9 |
| 15.37 | 48.2 | 27.66 | 1.7 |
| 16.20 | 10.1 | 28.17 | 2.0 |
| 16.43 | 15.4 | 28.63 | 6.6 |
| 16.68 | 3.8 | 28.88 | 2.6 |
| 17.87 | 11.0 | 29.67 | 1.3 |
| 18.04 | 15.7 | 30.29 | 4.0 |
| 19.07 | 9.7 | 31.32 | 1.9 |
| 19.34 | 27.8 | 32.33 | 1.6 |
| 19.59 | 8.2 | 32.72 | 2.2 |
| 20.04 | 3.7 | 33.00 | 1.4 |
| 20.46 | 23.1 | 34.36 | 1.9 |
| 21.15 | 8.7 | 34.82 | 1.2 |
| 21.63 | 8.4 | 35.26 | 2.1 |
| 22.01 | 7.4 | 35.98 | 1.8 |
| 22.65 | 3.8 | 36.29 | 1.5 |
| 23.12 | 12.0 | 39.17 | 3.6 |
| 23.42 | 3.0 | | |

Preferably, the crystal form N has X-ray powder diffraction intensities shown in Table 14.

Preferably, the crystal form N has an X-ray powder diffraction pattern substantially as shown in FIG. 32.

In a second aspect of the present disclosure, provided is a method for preparing the polymorph of compound I described above, comprising:
step 1: dissolving or dispersing compound I in a solvent; and
step 2: stirring at 0-50 °C for crystallization, or adding an antisolvent into a clarified solution of the compound for crystallization, or slowly evaporating the clarified solution of the compound.

As a further preferred embodiment, the solvent is water, an organic solvent, or a mixed solvent thereof. The organic solvent is selected from alcohols, chloroalkanes, ketones, ethers, cyclic ethers, esters, alkanes, cycloalkanes, benzenoids, amides, sulfoxides, and a mixture thereof; preferably, the organic solvent is selected from methanol, ethanol, n-propanol, isopropanol, n-butanol, trifluoroethanol, acetonitrile, acetone, methyl ethyl ketone, methyl isobutyl ketone, 1,4-dioxane, tetrahydrofuran, 2-methyltetrahydrofuran, N,N-dimethylformamide, N,N-dimethylacetamide, N-methylpyrrolidone, dimethylsulfoxide, ethyl acetate, isopropyl acetate, dichloromethane, trichloromethane, trichloroethane, tetrachloromethane, methyl *tert-butyl* ether, cyclopentyl methyl ether, 2-methoxyethyl ether, isopropyl ether, diethyl ether, n-heptane, n-hexane, isooctane, pentane, cyclohexane, cyclopentane, methylcyclohexane, benzene, toluene, xylene, and a mixture thereof.

In a third aspect of the present disclosure, provided is a pharmaceutical composition, which comprises at least one of the polymorphs of compound I described above and a pharmaceutically acceptable carrier.

In a fourth aspect of the present disclosure, provided is use of the polymorph of compound I described above for manufacturing a medicament for the treatment of a metabolic disease, a tumor, an autoimmune disease, or a metastatic disease.

In a fifth aspect of the present disclosure, provided is the polymorph of compound I described above for use as a medicament for the treatment of a metabolic disease, a tumor, an autoimmune disease, or a metastatic disease.

In a sixth aspect of the present disclosure, provided is the polymorph of compound I described above for use as a medicament for the prevention or treatment of T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, or polycystic ovary syndrome, and the treatment of addiction.

As a preferred embodiment, the polymorph of compound I described above is for use as a medicament for the treatment of T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, obesity, eating disorders, weight gain caused by use of other medicaments, excessive sugar craving, dyslipidemia, or hyperinsulinemia.

The present disclosure further provides a method for treating a disease, which comprises administering to an individual in need thereof a therapeutically effective amount of at least one of the polymorphs of compound I and the pharmaceutical composition described above.

According to the embodiments of the present disclosure, the disease is selected from a metabolic disease, a tumor, an autoimmune disease, and a metastatic disease.

According to the embodiments of the present disclosure, the disease is selected from T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, and polycystic ovary syndrome.

### Beneficial Effects

The present disclosure provides a polymorph of compound I and a method for preparing same. The polymorph has the advantages of good stability, good flowability, and easy pulverization, making it more suitable for the development of clinical formulations. The method provided by the present disclosure features simplified processes, ease of implementation, mild conditions for reaction, and high product yields. Moreover, the present disclosure does not require excessive purification procedures and features safe and environment-friendly operations, thus favoring the industrial production of polymorphs. The present disclosure also meets the requirements of the development of clinical pharmaceutical formulations, has very important clinical application value, and is expected to accelerate the development of a new-generation GLP-1R small-molecular agonist.

### BRIEF DESCRIPTION OF THE DRAWINGS

FIG. 1 illustrates the X-ray powder diffraction pattern of a crystal form A of compound I of the present disclosure. The abscissa denotes 2θ values (degrees), and the ordinate denotes peak intensity.
FIG. 2 illustrates the DSC profile of a crystal form A of compound I of the present disclosure. The abscissa denotes temperature (°C), and the ordinate denotes heat flow (mW).
FIG. 3 illustrates the TGA profile of a crystal form A of compound I of the present disclosure. The abscissa denotes temperature (°C), and the ordinate denotes weight (%).
FIG. 4 illustrates the X-ray powder diffraction pattern of a crystal form B of compound I of the present disclosure. The abscissa denotes 2θ values (degrees), and the ordinate denotes peak intensity.
FIG. 5 illustrates the DSC profile of a crystal form B of compound I of the present disclosure. The abscissa denotes temperature (°C), and the ordinate denotes heat flow (mW).
FIG. 6 illustrates the TGA profile of a crystal form B of compound I of the present disclosure. The abscissa denotes temperature (°C), and the ordinate denotes weight (%).
FIG. 7 illustrates the X-ray powder diffraction pattern of a crystal form C of compound I of the present disclosure. The abscissa denotes 2θ values (degrees), and the ordinate denotes peak intensity.
FIG. 8 illustrates the DSC profile of a crystal form C of compound I of the present disclosure. The abscissa denotes temperature (°C), and the ordinate denotes heat flow (mW).
FIG. 9 illustrates the TGA profile of a crystal form C of compound I of the present disclosure. The abscissa denotes temperature (°C), and the ordinate denotes weight (%).
FIG. 10 illustrates the X-ray powder diffraction pattern of a hydrate crystal form D of compound I of the present disclosure. The abscissa denotes 2θ values (degrees), and the ordinate denotes peak intensity.
FIG. 11 illustrates the DSC profile of a hydrate crystal form D of compound I of the present disclosure. The abscissa denotes temperature (°C), and the ordinate denotes heat flow (mW).
FIG. 12 illustrates the TGA profile of a hydrate crystal form D of compound I of the present disclosure. The abscissa denotes temperature (°C), and the ordinate denotes weight (%).
FIG. 13 illustrates the X-ray powder diffraction pattern of a trichloromethane solvate crystal form E of compound I of the present disclosure. The abscissa denotes 2θ values (degrees), and the ordinate denotes peak intensity.
FIG. 14 illustrates the DSC profile of a trichloromethane solvate crystal form E of compound I of the present disclosure. The abscissa denotes temperature (°C), and the ordinate denotes heat flow (mW).
FIG. 15 illustrates the TGA profile of a trichloromethane solvate crystal form E of compound I of the present disclosure. The abscissa denotes temperature (°C), and the ordinate denotes weight (%).
FIG. 16 illustrates the X-ray powder diffraction pattern of a N-methylpyrrolidone solvate crystal form F of compound I of the present disclosure. The abscissa denotes 2θ values (degrees), and the ordinate denotes peak intensity.
FIG. 17 illustrates the DSC profile of a N-methylpyrrolidone solvate crystal form F of compound I of the present disclosure. The abscissa denotes temperature (°C), and the ordinate denotes heat flow (mW).
FIG. 18 illustrates the TGA profile of a N-methylpyrrolidone solvate crystal form F of compound I of the present disclosure. The abscissa denotes temperature (°C), and the ordinate denotes weight (%).
FIG. 19 illustrates the X-ray powder diffraction pattern of a N-methylpyrrolidone solvate crystal form G of compound I of the present disclosure. The abscissa denotes 2θ values (degrees), and the ordinate denotes peak intensity.
FIG. 20 illustrates the DSC profile of a N-methylpyrrolidone solvate crystal form G of compound I of the present disclosure. The abscissa denotes temperature (°C), and the ordinate denotes heat flow (mW).
FIG. 21 illustrates the TGA profile of a N-methylpyrrolidone solvate crystal form G of compound I of the present disclosure. The abscissa denotes temperature (°C), and the ordinate denotes weight (%).
FIG. 22 illustrates the X-ray powder diffraction pattern of a tetrahydrofuran solvate crystal form H of compound I of the present disclosure. The abscissa denotes 2θ values (degrees), and the ordinate denotes peak intensity.
FIG. 23 illustrates the DSC profile of a tetrahydrofuran solvate crystal form H of compound I of the present disclosure. The abscissa denotes temperature (°C), and the ordinate denotes heat flow (mW).
FIG. 24 illustrates the TGA profile of a tetrahydrofuran solvate crystal form H of compound I of the present disclosure. The abscissa denotes temperature (°C), and the ordinate denotes weight (%).
FIG. 25 illustrates the X-ray powder diffraction pattern of an ethanol solvate crystal form I of compound I of the present disclosure. The abscissa denotes 2θ values (degrees), and the ordinate denotes peak intensity.
FIG. 26 illustrates the DSC profile of an ethanol solvate crystal form I of compound I of the present disclosure. The abscissa denotes temperature (°C), and the ordinate denotes heat flow (mW).
FIG. 27 illustrates the TGA profile of an ethanol solvate crystal form I of compound I of the present disclosure. The abscissa denotes temperature (°C), and the ordinate denotes weight (%).
FIG. 28 illustrates the X-ray powder diffraction pattern of a metastable crystal form J of compound I of the present disclosure. The abscissa denotes 2θ values (degrees), and the ordinate denotes peak intensity.
FIG. 29 illustrates the X-ray powder diffraction pattern of a metastable crystal form K of compound I of the present disclosure. The abscissa denotes 2θ values (degrees), and the ordinate denotes peak intensity.
FIG. 30 illustrates the X-ray powder diffraction pattern of a metastable crystal form L of compound I of the present disclosure. The abscissa denotes 2θ values (degrees), and the ordinate denotes peak intensity.
FIG. 31 illustrates the X-ray powder diffraction pattern of a metastable crystal form M of compound I of the present disclosure. The abscissa denotes 2θ values (degrees), and the ordinate denotes peak intensity.
FIG. 32 illustrates the X-ray powder diffraction pattern of a metastable crystal form N of compound I of the present disclosure. The abscissa denotes 2θ values (degrees), and the ordinate denotes peak intensity.
FIG. 33 illustrates the DVS plot of a crystal form A of compound I of the present disclosure. The abscissa denotes relative humidity (%), and the ordinate denotes weight change (%).

### Definitions and Description

Unless otherwise stated, the following terms used in the specification and the claims have the following meanings. A particular phrase or term, unless otherwise specifically defined, should not be considered uncertain or unclear, but construed according to its common meaning. When referring to a trade name, it is intended to refer to its corresponding commercial product or its active ingredient.

The "pharmaceutical composition" refers to a mixture containing one or more of the compounds described herein or a physiologically/pharmaceutically acceptable salt or prodrug thereof and other chemical components, as well as other components such as physiological/pharmaceutically acceptable carriers and excipients. The purpose of the pharmaceutical composition is to facilitate the administration to an organism, promote the absorption of the active ingredient, and thereby exert biological activity.

As used herein, the "polymorph" refers to crystal forms having the same chemical composition but different spatial arrangements of molecules, atoms, and/or ions composing the crystal. Although polymorphs have the same chemical composition, they differ in packing and geometric arrangement and may exhibit different physical properties such as melting point, shape, color, density, hardness, deformability, stability, solubility, dissolution rate, and the like. Two polymorphs may be monotropic or enantiotropic, depending on their temperature-stability relationship. For a monotropic system, the relative stability between the two solid phases remains unchanged upon temperature change. In contrast, in an enantiotropic system, there is a transition temperature at which the two phases are switched in stability ((Theory and Origin of Polymorphism in "Polymorphism in Pharmaceutical Solids" (1999) ISBN: )-8247-0237). The phenomenon that such compounds exist in different crystal structures is called polymorphism.

The various crystal structures of the present disclosure can be distinguished from one another using various analytical techniques known to those of ordinary skill in the art.

Such techniques include, but are not limited to, X-ray powder diffraction (XRPD), differential scanning calorimetry (DSC), and/or thermogravimetric analysis (TGA). The term "room temperature" or "RT" as used herein refers to an ambient temperature of 20 to 25 °C (68-77 °F).

The term "substantially identical" as used herein with respect to X-ray diffraction peak positions is meant to account for typical peak positions and intensity variability. For example, those skilled in the art will appreciate that the measured value of peak position (2θ) may vary due to different XRPD systems, sometimes by up to 0.2°. Furthermore, those skilled in the art will appreciate that factors such as XRPD sample preparation, XRPD system, sample crystallinity, sample amount, and preferred crystal orientation will result in variations in the relative peak intensities in the XRPD diffraction patterns of the samples.

The intermediate compounds of the present disclosure can be prepared by a variety of synthetic methods well known to those skilled in the art, including the specific embodiments listed below, embodiments formed by combinations thereof with other chemical synthetic methods, and equivalent substitutions thereof known to those skilled in the art. Preferred embodiments include but are not limited to the examples of the present disclosure.

The chemical reactions of the specific embodiments of the present disclosure are carried out in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction scheme based on the existing embodiments.

The present disclosure is described in detail below through examples; however, these examples are not intended to limit the present disclosure in any way.

All solvents used in the present disclosure are commercially available and can be used without further purification.

Unless otherwise stated, all reactions in the present disclosure were performed under continuous magnetic stirring in dry solvents, and the temperatures are expressed in degrees Celsius (°C).

### Methods and Materials

The structures of the compounds were determined by nuclear magnetic resonance (NMR). The NMR shifts (δ) are given in parts per million (ppm). The NMR analyses were performed using the Bruker avance-400MHz NMR spectrometer with deuterated dimethylsulfoxide (DMSO-*d₆*) or deuterated methanol (MeOD-*d₄*) as the solvent and tetramethylsilane (TMS) as the internal standard, and the chemical shifts are expressed in 10⁻⁶ ppm.

The HPLC procedures were performed using the Agilent 1260 high performance liquid chromatograph or an equivalent high performance liquid chromatograph (with a Sunfire C18 150 × 4.6 m chromatography column or an equivalent chromatography column).

The polymorphs of compound I were characterized by X-ray powder diffraction patterns. The X-ray powder diffraction patterns of the salts were collected on the Bruker D8 Advance powder diffractometer operating in reflection mode using Cu Kα radiation. The instrument used Cu Kα radiation (40 kV, 40 mA) and operated at room temperature using the SSD160-2 detector. The 2θ scan range was from 3° to 40°, and the scan speed was 0.1 s/step. The diffraction patterns were analyzed using the DIFFRAC.MEA.CENTER software.

XRPD samples were prepared by placing a sample on a monocrystal silicon wafer and pressing the sample powder with a glass slide or an equivalent to ensure that the sample had a flat surface and an appropriate height. The sample holder was then placed into the Bruker D8 Advance system, and the X-ray powder diffraction pattern was collected using the instrumental parameters described above. Measurement differences associated with such X-ray powder diffraction analysis results may result from a variety of factors including: (a) errors in sample preparation (e.g., sample height), (b) instrument errors, (c) calibration errors, (d) operator errors (including those errors produced when determining the peak positions), and (e) the nature of the material (e.g., preferred orientation errors). Calibration errors and sample height errors often result in a shift of all the peaks in the same direction. In general, this correction factor will bring the measured peak positions into agreement with the expected peak positions and may be in the range of the expected 2θ value±0.2°.

The experimental method for characterizing a crystal form of an acid salt or a base salt of compound I by differential scanning calorimetry (DSC) was as follows: A small amount of a polymorph of compound I in the crystal form was taken and placed into an aluminum crucible that matches the instrument and can be sealed with a lid. After the sample was loaded, the crucible was sealed with an aluminum plate and then sent into the instrument for analysis. In this patent, the instrument model used in the differential scanning calorimetry analysis was METTLER TOLEDO DSC 3, and scan parameters were set as follows: A nitrogen atmosphere was used, and the temperature ramp rate was 10.0 k/min.

The experimental method for characterizing a polymorph of compound I by thermogravimetric analysis (TGA) was as follows: A small amount of a powder of a polymorph of compound I was taken and placed into an aluminum oxide crucible that matches the instrument. After the sample was loaded, the crucible was sent into the instrument for analysis. In this patent, the instrument model used in the differential scanning calorimetry analysis was METTLER TOLEDO TGA 2, and scan parameters were set as follows: A nitrogen atmosphere was used, and the temperature ramp rate was 10.0 k/min.

The experimental method for characterizing an acid salt or a base salt of compound I by dynamic vapor sorption (DVS) was as follows: A small amount of a powder of a polymorph of compound I was taken and placed into a precise sample tray that matches the instrument. After the sample was loaded, the sample tray was sent into the instrument for analysis. In this patent, the instrument model used in the dynamic vapor sorption analysis was Intrinsic PLUS; the experimental parameter was set as follows: A constant temperature of 25 °C was set; the mass percent change rate per unit time (dm/dt) = 0.02%/min was used as a criterion for determining that a balance is achieved; the program humidity change cycle was set as follows: The initial relative humidity was 0%, and the endpoint relative humidity was 90%.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure. Unless otherwise stated, the starting materials and reagents used in the following examples are all commercially available products or can be prepared by using known methods.

### Example 1. Preparation of (S)-2-((4-((6-((4-chloro-2-fluorophenoxy)methyl)pyridin-2-yl)oxy)piperidin-1-yl)methyl)-1-(oxetan-2-ylmethyl)-1H-benzo[d]imidazole-6-carboxylic acid (free form of compound I)

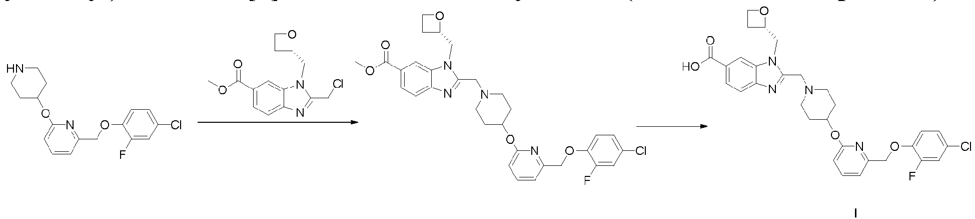

### Step 1: Synthesis of methyl (S)-2-((4-((6-((4-chloro-2-fluorophenoxy)methyl)pyridin-2-yl)oxy)piperidin-1-yl)methyl)-1-(oxetan-2-ylmethyl)-1H-benzo[d]imidazole-6-carboxylate

A mixed solution of methyl (*S*)-2-(chloromethyl)-1-(oxetan-2-ylmethyl)-1*H-*benzo[d]imidazole-6-carboxylate (1.5 g, 5.1 mmol), 2-((4-chloro-2-fluorophenoxy)methyl)-6-(piperidin-4-yloxy)pyridine (1.8 g, 5.5 mmol), and potassium carbonate (1.8 g, 13.0 mmol) in N,N-dimethylformamide (80 mL) was stirred at 60 °C for 3 h, then quenched with water (100 mL), and extracted with ethyl acetate (50 mL × 3). The combined organic layers were washed with brine (50 mL × 3), dried over anhydrous sodium sulfate, filtered, and concentrated. The residue was purified by silica gel column chromatography (dichloromethane/methanol = 20/1) to give methyl (*S*)-2-((4-((6-((4-chloro-2-fluorophenoxy)methyl)pyridin-2-yl)oxy)piperidin-1-yl)methyl)-1-(oxetan-2-ylmethyl)-1*H*-benzo[*d*]imidazole-6-carboxylate (1.0 g, 33.5% yield).

### Step 2: Synthesis of (S)-2-((4-((6-((4-chloro-2-fluorophenoxy)methyl)pyridin-2-yl)oxy)piperidin-1-yl)methyl)-1-(oxetan-2-ylmethyl)-1H-benzo[d]imidazole-6-carboxylic acid

Lithium hydroxide (0.13 g, 5.4 mmol) was added to a mixed solution of methyl (*S*)-2-((4-((6-((4-chloro-2-fluorophenoxy)methyl)pyridin-2-yl)oxy)piperidin-1-yl)methyl)-1-(oxetan-2-ylmethyl)-1*H*-benzo[d]imidazole-6-carboxylate (1.0 g, 1.7 mmol) in tetrahydrofuran/water (20 mL/20 mL), and the mixture was stirred at room temperature for 16 h. The pH of the resulting mixture was adjusted to 5-6 with formic acid, and the solvent was removed under vacuum. The residue was purified by reverse-phase flash column chromatography to give (*S*)-2-((4-((6-((4-chloro-2-fluorophenoxy)methyl)pyridin-2-yl)oxy)piperidin-1-yl)methyl)-1-(oxetan-2-ylmethyl)-1*H*-benzo[*d*]imidazole-6-carboxylic acid (0.69 g, 70.5% yield). ¹H NMR (400 MHz, DMSO-*d₆*): δ 8.27 (s, 1 H), 7.80 (dd, *J*=8.4 Hz, 1.2 Hz, 1 H), 7.72 (t, *J*=7.6 Hz, 1 H), 7.64 (d, *J*=8.4 Hz, 1 H), 7.44 (dd, *J*=11.2 Hz, 2.0 Hz, 1 H), 7.28 (t, *J*=8.8 Hz, 1 H), 7.18 (d, *J*=8.4 Hz, 1 H), 7.04 (d, *J*=7.2 Hz, 1 H), 6.72 (d, *J*=8.0 Hz, 1 H), 5.18 (s, 2 H), 5.12-5.06 (m, 1 H), 4.95-4.93 (m, 1 H), 4.81-4.76 (m, 1 H), 4.66-4.62 (m, 1 H), 4.51-4.49 (m, 1 H), 4.38-4.36 (m, 1 H), 3.94 (d, *J*=13.6 Hz, 1 H), 3.78 (d, *J*=13.6 Hz, 1 H), 2.79-2.67 (m, 2 H), 2.46-2.41 (m, 1 H), 2.32 (s, 2 H), 1.92-1.91 (m, 2 H), 1.63-1.59 (m, 2 H).

### Example 2. Preparation of Crystal Form A of Compound I

200 mg of the free form of compound I and 41.88 mg of tromethamine were added to 10 mL of isopropanol. The mixture was stirred at room temperature for 3 d and filtered. The filter cake was dried in an oven at 40 °C to give crystal form A of the compound. The product was analyzed and characterized by XRPD (FIG. 1), DSC (FIG. 2), and TGA (FIG. 3). ¹H NMR (400 MHz, CD₃OD): δ 8.20 (s, 1 H), 7.94 (dd, *J*=8.4 Hz, 1.2 Hz, 1 H), 7.67-7.57 (m, 2 H), 7.20-7.03 (m, 4 H), 6.66 (d, *J*=8.0 Hz, 1 H), 5.28-5.26 (m, 1H), 5.13 (s, 2 H), 5.05-5.03 (m, 1 H), 4.90-4.86 (m, 1 H), 4.73-4.62 (m, 2 H), 4.47-4.45 (m, 1 H), 4.02-3.89 (m, 2 H), 3.64 (s, 6 H), 2.81-2.77 (m, 3 H), 2.52-2.42 (m, 3 H), 2.01-1.99 (m, 2 H), 1.78-1.76 (m, 2 H).

### Example 3. Preparation of Crystal Form B of Compound I

200 mg of crystal form A of compound I was suspended in 11 mL of methanol/methyl *tert-butyl* ether (7:4, v: v). The mixture was stirred at 5 °C for 16 h and filtered. The filter cake was dried in an oven at 50 °C to give crystal form B of the compound. The product was analyzed and characterized by XRPD (FIG. 4), DSC (FIG. 5), and TGA (FIG. 6). ¹H NMR (400 MHz, CD₃OD): δ 8.20 (s, 1 H), 7.94 (dd, *J*=8.4 Hz, 1.2 Hz, 1 H), 7.67-7.57 (m, 2 H), 7.21-7.03 (m, 4 H), 6.67 (d, *J*=8.0 Hz, 1 H), 5.28-5.27 (m, 1H), 5.13 (s, 2 H), 5.05-5.03 (m, 1 H), 4.90-4.88 (m, 1 H), 4.73-4.62 (m, 2 H), 4.47-4.45 (m, 1 H), 4.02-3.89 (m, 2 H), 3.64 (s, 6 H), 2.83-2.77 (m, 3 H), 2.51-2.39 (m, 3 H), 2.01-1.99 (m, 2 H), 1.77-1.76 (m, 2 H).

### Example 4. Preparation of Crystal Form C of Compound I

20 mg of crystal form A of compound I was dissolved in 1 mL of methanol. The mixture was filtered, and 2 mg of copovidone was added to the filtrate. The resulting mixture was let stand at room temperature to slowly evaporate the solvent. The resulting solid was dried at 50 °C to give crystal form C of the compound. The product was analyzed and characterized by XRPD (FIG. 7), DSC (FIG. 8), and TGA (FIG. 9).

### Example 5. Preparation of Hydrate Crystal Form D of Compound I

200 mg of crystal form A of compound I was added to 9 mL of acetonitrile/water (6:1, v: v). The mixture was stirred at 5 °C for 24 h and filtered. The filter cake was dried in an oven at 50 °C to give crystal form D of the compound. The product was analyzed and characterized by XRPD (FIG. 10), DSC (FIG. 11), and TGA (FIG. 12). ¹H NMR (400 MHz, CD₃OD): δ 8.20 (s, 1 H), 7.94 (dd, *J*=8.5 Hz, 1.2 Hz, 1 H), 7.66-7.57 (m, 2 H), 7.20-7.03 (m, 4 H), 6.66 (d, *J*=8.1 Hz, 1 H), 5.27-5.26 (m, 1H), 5.13 (s, 2 H), 5.05-5.03 (m, 1 H), 4.90-4.85 (m, 1 H), 4.73-4.62 (m, 2 H), 4.47-4.45 (m, 1 H), 4.02-3.89 (m, 2 H), 3.64 (s, 6 H), 2.81-2.77 (m, 3 H), 2.52-2.39 (m, 3 H), 2.00-1.98 (m, 2 H), 1.78-1.76 (m, 2 H).

### Example 6. Preparation of Trichloromethane Solvate Crystal Form E of Compound I

20 mg of crystal form A of compound I was suspended in 1.4 mL of trichloromethane/trifluoroethanol (6:1, v:v). The mixture was stirred at 5 °C for 16 h and filtered. The filter cake was dried in an oven at 50 °C to give crystal form E of the compound. The product was analyzed and characterized by XRPD (FIG. 13), DSC (FIG. 14), and TGA (FIG. 15). ¹H NMR (400 MHz, CD₃OD): δ 8.20 (s, 1 H), 7.94 (dd, *J*=8.4 Hz, 1.2 Hz, 1 H), 7.90 (s, 0.8 H), 7.67-7.57 (m, 2 H), 7.21-7.03 (m, 4 H), 6.66 (d,*J*=8.0 Hz, 1 H), 5.28-5.26 (m, 1H), 5.13 (s, 2 H), 5.05-5.03 (m, 1 H), 4.90-4.86 (m, 1 H), 4.73-4.62 (m, 2 H), 4.47-4.45 (m, 1 H), 4.02-3.89 (m, 2 H), 3.64 (s, 6 H), 2.81-2.77 (m, 3 H), 2.54-2.42 (m, 3 H), 2.00-1.98 (m, 2 H), 1.78-1.76 (m, 2 H).

### Example 7. Preparation of N-Methylpyrrolidone Solvate Crystal Form F of Compound I

20 mg of crystal form A of compound I was suspended in 0.5 mL of *N-*methylpyrrolidone/cyclopentyl methyl ether (1:5, v:v). The mixture was stirred at 5 °C for 3 d and filtered. The filter cake was dried in an oven at 50 °C to give crystal form F of the compound. The product was analyzed and characterized by XRPD (FIG. 16), DSC (FIG. 17), and TGA (FIG. 18). ¹H NMR (400 MHz, CD₃OD): δ 8.20 (s, 1 H), 7.94 (dd, *J*=8.4 Hz, 1.2 Hz, 1 H), 7.67-7.57 (m, 2 H), 7.21-7.03 (m, 4 H), 6.67 (d, *J*=8.0 Hz, 1 H), 5.28-5.25 (1H), 5.13 (s, 2 H), 5.05-5.03 (m, 1 H), 4.90-4.88 (m, 1H), 4.73-4.62 (m, 2 H), 4.47-4.45 (m, 1 H), 4.02-3.89 (m, 2 H), 3.64 (s, 6 H), 3.46-3.42 (m, 3.7 H), 2.82-2.80 (m, 8.5 H), 2.51-2.33 (m, 7 H), 2.07-1.99 (m, 5.7 H), 1.78-1.76 (m, 2 H).

### Example 8. Preparation of N-Methylpyrrolidone Solvate Crystal Form G of Compound I

200 mg of crystal form A of compound I was dissolved in 2.0 mL of *N-*methylpyrrolidone, and the solution was dropwise added to 15 mL of toluene. The mixture was stirred at room temperature for 16 h and filtered. The filter cake was dried in an oven at 50 °C to give crystal form G of the compound. The product was analyzed and characterized by XRPD (FIG. 19), DSC (FIG. 20), and TGA (FIG. 21). ¹H NMR (400 MHz, CD₃OD): δ 8.20 (s, 1 H), 7.94 (dd, J=8.4 Hz, 1.2 Hz, 1 H), 7.66-7.57 (m, 2 H), 7.21-7.03 (m, 4 H), 6.66 (d, J=8.0 Hz, 1 H), 5.28-5.25 (1H), 5.12 (s, 2 H), 5.05-5.03 (m, 1 H), 4.73-4.62 (m, 2 H), 4.47-4.45 (m, 1 H), 4.02-3.88 (m, 2 H), 3.65 (s, 6 H), 3.44 (d, J=7.2 Hz, 3.5 H), 2.82-2.77 (m, 8 H), 2.51-2.33 (m, 6.5 H), 2.07-1.99 (m, 5.5 H), 1.77-1.76 (m, 2 H).

### Example 9. Preparation of Tetrahydrofuran Solvate Crystal Form H of Compound I

20 mg of crystal form A of compound I was suspended in 0.5 mL of tetrahydrofuran/water (9:1, v:v). The mixture was stirred at 50 °C for 2 h, cooled to 5 °C, stirred for 2 h, and filtered. The filter cake was dried in an oven at 50 °C to give crystal form H of the compound. The product was analyzed and characterized by XRPD (FIG. 22), DSC (FIG. 23), and TGA (FIG. 24). ¹H NMR (400 MHz, CD₃OD): δ 8.20 (s, 1 H), 7.94 (dd, *J*=8.4 Hz, 1.2 Hz, 1 H), 7.67-7.57 (m, 2 H), 7.21-7.03 (m, 4 H), 6.67 (d, *J*=8.0 Hz, 1 H), 5.28-5.25 (1H), 5.13 (s, 2 H), 5.05-5.03 (m, 1 H), 4.73-4.62 (m, 2 H), 4.46-4.44 (m, 1 H), 4.02-3.89 (m, 2 H), 3.74-3.71 (m, 1.5 H), 3.64 (s, 6 H), 2.81-2.77 (m, 3 H), 2.53-2.42 (m, 3 H), 2.07-1.99 (m, 2 H), 1.88-1.85 (m, 1.5 H), 1.77-1.75 (m, 2 H).

### Example 10. Preparation of Ethanol Solvate Crystal Form I of Compound I

100 mg of crystal form A of compound I was suspended in 5 mL of ethanol/water (98:2, v:v). The mixture was stirred at 5 °C for 16 h and filtered. The filter cake was dried in an oven at 50 °C to give crystal form I of the compound. The product was analyzed and characterized by XRPD (FIG. 25), DSC (FIG. 26), and TGA (FIG. 27). ¹H NMR (400 MHz, CD₃OD): δ 8.20 (s, 1 H), 7.94 (dd, *J*=8.4 Hz, 1.2 Hz, 1 H), 7.67-7.57 (m, 2 H), 7.21-7.03 (m, 4 H), 6.66 (d, *J*=8.0 Hz, 1 H), 5.27-5.25 (m, 1H), 5.13 (s, 2 H), 5.05-5.03 (m, 1 H), 4.90-4.88 (m, 1 H), 4.73-4.62 (m, 2 H), 4.47-4.45 (m, 1 H), 4.02-3.89 (m, 2 H), 3.63 (s, 6 H), 3.60 (q, *J*=8.0 Hz, 1.8 H), 2.81-2.77 (m, 3 H), 2.52-2.42 (m, 3 H), 2.01-1.99 (m, 2 H), 1.78-1.76 (m, 2 H), 1.17 (t, *J*=8.0 Hz, 2.6 H).

### Example 11. Preparation of Metastable Crystal Form J of Compound I

200 mg of the free form of compound I and 42 mg of tromethamine were added to 10 mL of IPA to form a suspension. After being stirred at room temperature for about 3 d, the mixture was filtered under vacuum. The resulting filter cake was metastable crystal form J of compound I. The product was analyzed and characterized by XRPD (FIG. 28). After being dried at 40 °C for 16 h, crystal form J was transformed to crystal form A.

### Example 12. Preparation of Metastable Crystal Form K of Compound I

100 mg of crystal form A of compound I was dissolved in 1.0 mL of DMF, and the solution was dropwise added to 15 mL of acetone. The mixture was stirred at room temperature for 16 h and filtered. The resulting filter cake was metastable crystal form K of compound I. The product was analyzed and characterized by XRPD (FIG. 29). After being dried at 50 °C for 24 h, crystal form K was transformed to crystal form A.

### Example 13. Preparation of Metastable Crystal Form L of Compound I

100 mg of crystal form A of compound I was suspended in 2.5 mL of H₂O/EtOH (1:9, v:v). The mixture was stirred at room temperature for about 3 d and filtered. The filter cake was dried at 50 °C for 16 h to give metastable crystal form L of compound I. The product was analyzed and characterized by XRPD (FIG. 30). After being let stand in an open flask at room temperature for about 16 h, crystal form L was transformed to crystal form D.

### Example 14. Preparation of Metastable Crystal Form M of Compound I

200 mg of crystal form A of compound I was suspended in 9 mL of ACN/H₂O (6:1, v:v). The mixture was stirred at 5 °C for about 1 d and filtered. The resulting filter cake was metastable crystal form M of compound I. The product was analyzed and characterized by XRPD (FIG. 31). After being dried under vacuum at room temperature for about 16 h, crystal form L was transformed to crystal form D.

### Example 15. Preparation of Metastable Crystal Form N of Compound I

Crystal form C of compound I was heated to 80 °C at 10 °C/min to give metastable crystal form N of compound I. The product was analyzed and characterized by XRPD (FIG. 32). After being dried at 50 °C for 16 h, crystal form N was transformed to crystal form B.

### Test Example 1. Equilibrium Solubility Study of Polymorphs of Compound I in Biological Media

The free form of compound I and crystal form A of compound I were tested for their equilibrium solubilities in water (H₂O), fasted state simulated gastric fluid (FaSSGF), fasted state simulated intestinal fluid (FaSSIF), and fed state simulated intestinal fluid (FeSSIF). In the tests, the solids were suspended in corresponding buffers to formulate suspensions (~10 mg/mL), and the suspensions were mixed at 37±2 °C. After 24 h, samples of the suspensions were taken. The supernatant was filtered, and the concentration was determined. The test results are shown in the following table:

| Solid form | Preparation method | Solution | Equilibrium solubility |
|---|---|---|---|
| Free form of compound I | Example 1 | H₂O | 0.19 mg/mL |
| | | FaSSIF | 0.05 mg/mL |
| | | FeSSIF | 0.16 mg/mL |
| Crystal form A of compound I | Example 2 | H₂O | 0.44 mg/mL |
| | | FaSSIF | 0.11 mg/mL |
| | | FeSSIF | 0.45 mg/mL |

It can be seen from the above results that the solubility of crystal form A of compound I in several vehicles is significantly superior to that of the free form of compound I and can meet the requirement of the development of clinical pharmaceutical formulations. Therefore, after salification of the free form of compound I, the solubility and the drug release behavior can be significantly improved.

### Test Example 2. Solid State Stability Study of Polymorphs of Compound I

The free form of compound I and crystal form A of compound I were preserved in long-term storage (25 °C/60% RH), accelerated (40 °C/75% RH), and high-temperature (60 °C, RH < 30%) conditions for 7 days and tested for purity and crystal form change by HPLC, so as to examine the stability of the solids. The results are shown in the following table:

| Solid form | Initial purity (%) | 25 °C/60% RH, 7 days | | 40 °C/75% RH, 7 days | | 60 °C, 7 days | |
|---|---|---|---|---|---|---|---|
| | | Purity (%) | Crystal form | Purity (%) | Crystal form | Purity (%) | Crystal form |
| Free form of compound I | 98.98 | 98.94 | Amorphous form | 95.86 | Amorphous form | 89.15 | Amorphous form |
| Crystal form A of compound I | 99.30 | 99.18 | Crystal form A | 99.17 | Crystal form A + crystal form D | 98.83 | Crystal form A |
| Crystal form B of compound I | 98.80 | 98.74 | Crystal form B | 98.73 | Crystal form B | 98.67 | Crystal form B |
| Crystal form I of compound I | 98.44 | 98.41 | Crystal form I + crystal form B | 98.47 | Crystal form I + crystal form B | 98.48 | Crystal form I + crystal form B |

It can be seen from the above purity results that the free form of compound I exhibited no significant purity changes after standing for 7 days in the long-term storage condition, but significant degradation after standing for 7 days in the accelerated condition and the high-temperature condition. Crystal form A, crystal form B, and crystal form I of compound I exhibited no significant purity changes after standing for 7 days in the long-term storage condition, the accelerated condition, and the high-temperature condition, and the stability is significantly superior to that of the free form. It can be seen from the crystal form results that crystal form A exhibited no crystal form changes after standing for 7 days in the long-term storage condition and the high-temperature condition, and part of crystal form A was converted into crystal form D after standing for 7 days in the accelerated condition. Crystal form B exhibited no crystal form changes after standing for 7 days in the long-term storage condition, the accelerated condition, and the high-temperature condition. Crystal form I exhibited crystal form changes after standing for 7 days in the long-term storage condition, the accelerated condition, and the high-temperature condition, and part of crystal form I was converted into crystal form B.

### Test Example 3. Competitive Experimental Study of Polymorphs of Compound I

Crystal form A and crystal form B of compound I were subjected to suspension competition experiments in acetonitrile (at room temperature and 50 °C) and ethyl acetate (at room temperature and 50 °C), separately, to test for crystal form changes. The results are shown in the following table:

| Starting crystal form | Solvent | Temperature | 1-Day result | 4-Day result | 7-Day result |
|---|---|---|---|---|---|
| Physical mixture of crystal form A and crystal form B | Acetonitrile | Room temperature | Crystal form A | N/A | N/A |
| | | 50°C | Crystal form A | N/A | N/A |
| | Ethyl acetate | Room temperature | Crystal form A + crystal form B | Crystal form A + crystal form B | Crystal form A + crystal form B |
| | | 50°C | Crystal form A + crystal form B | Crystal form A | N/A |

It can be seen from the above results that crystal form B of compound I was converted into crystal form A in both acetonitrile (at room temperature and 50 °C) and ethyl acetate (50 °C) systems. In the ethyl acetate system at room temperature, after being stirred for 7 days, the mixture remained as a mixed crystal of crystal form A and crystal form B, but the trend that crystal form B was converted into crystal form A can be observed, indicating that within the temperature range from room temperature to 50 °C, crystal form A exhibits higher thermodynamic stability than crystal form B.

### Test Example 4. Hygroscopic Behavior Test of Compound I

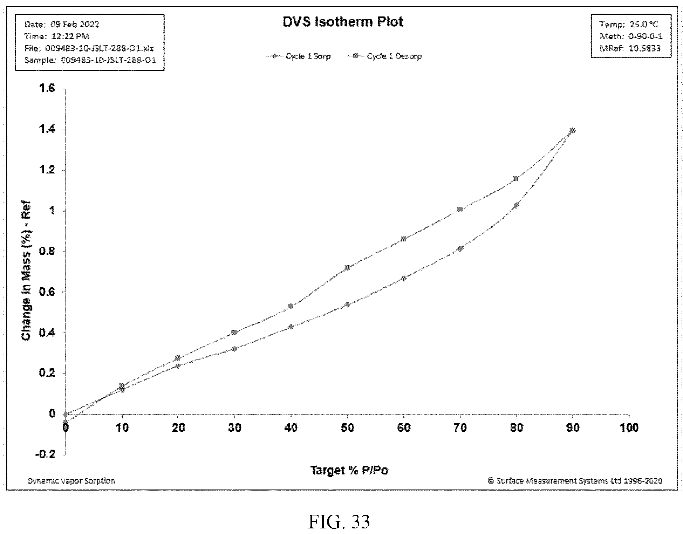

In this patent, the inventors assessed the stability risk of samples under varying humidity at 25 °C using the dynamic vapor sorption method and conducted the DVS test on the representative crystal form A of compound I to evaluate the hygroscopicity of the crystal form of the compound. The DVS plot of crystal form A of the compound is shown in FIG. 33. The results are shown in the following table:

| Solid form | Sample weight change at 80% RH (%) | Whether the crystal form is changed | Hygroscopicity |
|---|---|---|---|
| Crystal form A of compound I | 1.03 | No | Slightly hygroscopic |

It can be seen from the above results that on the 0-90% RH adsorption curves, at 80% RH, crystal form A of compound I was slightly hygroscopic, and no solid form changes were observed.

### Test Example 5. Drug Absorption Experiment in SD Rats

Intravenous administration to rats: Healthy SD rats were given the free form or crystal form A of compound I via tail vein injection at a dose of D mg/kg. Blood samples were collected at different time points before and after administration and then separated to prepare plasma. The concentration of the drug in plasma was determined by liquid chromatography-tandem mass spectrometry, and pharmacokinetic parameters were calculated using a non-compartmental model.

The main pharmacokinetic parameters are shown in the following table:

| Compound | Dose D (mg/kg) | Cₘₐₓ (ng/mL) | AUC₀₋ₜ (ng.h/mL) | t_{1/2} (h) |
|---|---|---|---|---|
| Free form of compound I | 1 | 3195 | 1281 | 3.89 |
| Crystal form A of compound I | 2 | 7640 | 2440 | 1.88 |

Intragastric administration to rats: Healthy SD rats were intragastrically given the free form or crystal form A of compound I, separately. Blood samples were collected at different time points after administration and then separated to prepare plasma. The concentration of the drug in plasma was determined by liquid chromatography-tandem mass spectrometry, and pharmacokinetic parameters were calculated using a non-compartmental model.

The main pharmacokinetic parameters are shown in the following table:

| Compound | Dose D (mg/kg) | Tₘₐₓ (h) | Cₘₐₓ (ng/mL) | AUC₀₋ₜ (ng.h/mL) | t_{1/2} (h) |
|---|---|---|---|---|---|
| Free form of compound I | 5 | 0.50 | 600 | 1395 | 2.84 |
| Crystal form A of compound I | 5 | 0.25 | 847 | 2670 | 3.59 |

After dose correction, the absolute bioavailability of the free form of compound I intragastrically administered was calculated to be 21.8% based on AUC₀₋ₜ. The absolute bioavailability of crystal form A intragastrically administered was 43.7%, which is significantly superior to that of the free form, indicating a pharmacokinetic advantage.

The thermal analysis of some of the crystal forms of compound I of the present disclosure is summarized in the following table:

| Crystal form | DSC endothermic peak temperature | TGA analysis | Solvent residue/solvate |
|---|---|---|---|
| Crystal form A | 174.82°C | 0.3514% weight loss at 30-120 °C | N/A |
| Crystal form B | 160.15°C | 0.3229% weight loss at 30-120 °C | N/A |
| Crystal form C | 160.99 °C and 170.25 °C | 0.3225% weight loss at 30-120 °C | N/A |
| Crystal form D | 62.09 °C, 79.77 °C, and 173.12 °C | 3.5026% weight loss at 30-120 °C | 1.5-hydrate |
| Crystal form E | 137.77 °C, 159.79 °C, and 175.44 °C | 11.4130% weight loss at 30-180 °C | Mono-trichloromethane solvate |
| Crystal form F | 72.65°C, 95.65°C, 127.81°C, 159.14°C | 20.1668% weight loss at 30-160 °C | Di-*N*-methylpyrrolidone solvate |
| Crystal form G | 109.95 °C and 166.02 °C | 18.8996% weight loss at 30-170 °C | 1.5-*N*-methylpyrrolidone solvate |
| Crystal form H | 82.80 °C and 174.84 °C | 4.5161% weight loss at 30-130 °C | 0.5-tetrahydrofuran solvate |
| Crystal form I | 136.10 °C and 160.74 °C | 5.5631% weight loss at 30-170 °C | Mono-ethanol solvate |

The embodiments of the technical solutions of the present disclosure have been described above by way of example. It will be appreciated that the claimed scope of the present disclosure is not limited to the embodiments described above. Any modification, equivalent substitution, improvement, and the like made by those skilled in the art without departing from the spirit and principle of the present disclosure shall fall within the claimed scope of the present application.

## Claims

1. A polymorph of compound I:

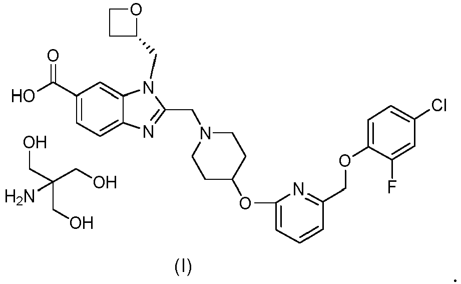

2. The polymorph according to claim 1, wherein the polymorph is a solvent-free crystal form of the compound of formula (I), including the following solvent-free crystal forms A, B, and C, wherein:
the crystal form A has an X-ray powder diffraction pattern comprising peaks at diffraction angles (2θ) of 7.42±0.2°, 3.68±0.2°, 21.85±0.2°, and 18.75±0.2°,
preferably further comprising peaks at diffraction angles (2θ) of 9.29±0.2°, 16.71±0.2°, 11.18±0.2°, 15.32±0.2°, and 14.94±0.2°, and
more preferably, further comprising peaks at diffraction angles (2θ) of 27.81±0.2°, 17.00±0.2°, 19.81±0.2°, 11.80±0.2°, 25.69±0.2°, and 17.43±0.2°;
preferably, the crystal form A has an X-ray powder diffraction pattern comprising diffraction angles (2θ) shown in Table 1, wherein the 2θ angles have a margin of error of ±0.20°:
**Table 1**
| 2θ (°) | Intensity% | 2θ (°) | Intensity% |
|---|---|---|---|
| 3.68 | 51.4 | 19.81 | 6.5 |
| 7.42 | 100.0 | 20.94 | 1.9 |
| 9.29 | 13.0 | 21.85 | 27.3 |
| 11.18 | 12.1 | 22.98 | 1.5 |
| 11.80 | 6.3 | 23.80 | 2.2 |
| 14.45 | 4.3 | 25.69 | 5.7 |
| 14.94 | 8.7 | 27.81 | 6.7 |
| 15.32 | 9.3 | 28.73 | 3.4 |
| 15.65 | 3.4 | 30.07 | 2.1 |
| 16.71 | 12.8 | 32.14 | 1.1 |
| 17.00 | 6.5 | 33.43 | 1.5 |
| 17.43 | 4.9 | 33.93 | 2.4 |
| 18.75 | 26.1 | | |
preferably, the crystal form A has X-ray powder diffraction intensities shown in Table 1;
preferably, the crystal form A has an X-ray powder diffraction pattern substantially as shown in FIG. 1;
preferably, the crystal form A exhibits an endothermic peak in a DSC analysis when heated to a peak temperature of about 174.82 °C;
preferably, the crystal form A has a DSC profile substantially as shown in FIG. 2;
preferably, the crystal form A has a TGA profile substantially as shown in FIG. 3;
the crystal form B has an X-ray powder diffraction pattern comprising peaks at diffraction angles (2θ) of 7.07±0.2°, 19.25±0.2°, 14.45±0.2°, and 15.26±0.2°,
preferably further comprising peaks at diffraction angles (2θ) of 11.20±0.2°, 18.17±0.2°, 13.26±0.2°, 23.28±0.2°, and 21.35±0.2°, and
more preferably, further comprising peaks at diffraction angles (2θ) of 15.72±0.2°, 26.17±0.2°, 16.54±0.2°, 24.23±0.2°, 14.76±0.2°, and 22.65±0.2°;
preferably, the crystal form B has an X-ray powder diffraction pattern comprising diffraction angles (2θ) shown in Table 2, wherein the 2θ angles have a margin of error of ±0.20°:
**Table 2**
| 2θ (°) | Intensity% | 2θ (°) | Intensity% |
|---|---|---|---|
| 5.58 | 2.4 | 22.65 | 7.3 |
| 7.07 | 100.0 | 23.28 | 11.3 |
| 9.02 | 2.1 | 23.72 | 4.0 |
| 10.81 | 6.4 | 24.23 | 7.8 |
| 11.20 | 13.0 | 24.62 | 2.7 |
| 12.43 | 0.9 | 25.18 | 1.4 |
| 13.26 | 12.4 | 25.65 | 6.6 |
| 14.18 | 1.9 | 26.17 | 8.9 |
| 14.45 | 33.7 | 26.67 | 3.2 |
| 14.76 | 7.5 | 27.01 | 2.0 |
| 15.26 | 27.0 | 27.33 | 1.6 |
| 15.56 | 2.3 | 27.51 | 1.5 |
| 15.72 | 9.4 | 28.23 | 2.7 |
| 16.54 | 7.8 | 29.18 | 5.1 |
| 16.77 | 4.0 | 30.45 | 6.4 |
| 17.51 | 2.3 | 30.86 | 2.1 |
| 18.17 | 12.7 | 31.50 | 0.7 |
| 18.64 | 0.7 | 32.01 | 1.2 |
| 19.25 | 38.8 | 32.83 | 0.8 |
| 19.54 | 6.1 | 33.56 | 2.7 |
| 19.75 | 2.6 | 33.94 | 2.4 |
| 20.07 | 0.8 | 35.00 | 1.7 |
| 20.53 | 4.4 | 36.43 | 1.4 |
| 20.96 | 3.3 | 37.05 | 2.5 |
| 21.35 | 10.3 | 37.87 | 0.6 |
| 22.09 | 5.3 | 38.79 | 2.0 |
| 22.32 | 5.4 | | |
preferably, the crystal form B has X-ray powder diffraction intensities shown in Table 2;
preferably, the crystal form B has an X-ray powder diffraction pattern substantially as shown in FIG. 4;
preferably, the crystal form B exhibits an endothermic peak in a DSC analysis when heated to a peak temperature of about 160.15 °C;
preferably, the crystal form B has a DSC profile substantially as shown in FIG. 5;
preferably, the crystal form B has a TGA profile substantially as shown in FIG. 6;
the crystal form C has an X-ray powder diffraction pattern comprising peaks at diffraction angles (2θ) of 10.90±0.2°, 5.80±0.2°, 19.28±0.2°, and 14.49±0.2°,
preferably further comprising peaks at diffraction angles (2θ) of 15.37±0.2°, 12.95±0.2°, 19.77±0.2°, 3.11±0.2°, and 24.46±0.2°, and
more preferably, further comprising peaks at diffraction angles (2θ) of 16.38±0.2°, 25.22±0.2°, 19.03±0.2°, 20.82±0.2°, 18.00±0.2°, and 18.15±0.2°;
preferably, the crystal form C has an X-ray powder diffraction pattern comprising diffraction angles (2θ) shown in Table 3, wherein the 2θ angles have a margin of error of ±0.20°:
**Table 3**
| 2θ (°) | Intensity% | 2θ (°) | Intensity% |
|---|---|---|---|
| 3.11 | 16.8 | 19.77 | 20.1 |
| 3.60 | 11.0 | 20.02 | 8.5 |
| 5.40 | 11.2 | 20.45 | 7.5 |
| 5.80 | 29.1 | 20.82 | 13.6 |
| 6.91 | 6.1 | 21.08 | 9.4 |
| 8.07 | 7.9 | 21.42 | 8.2 |
| 8.80 | 7.6 | 21.99 | 10.1 |
| 9.00 | 6.0 | 22.62 | 9.0 |
| 9.29 | 3.4 | 22.77 | 9.1 |
| 9.88 | 9.1 | 23.20 | 12.0 |
| 10.90 | 100.0 | 23.93 | 6.8 |
| 12.46 | 4.4 | 24.21 | 12.0 |
| 12.95 | 23.8 | 24.46 | 16.6 |
| 13.57 | 10.8 | 24.87 | 3.6 |
| 13.79 | 3.4 | 25.22 | 14.3 |
| 14.18 | 7.2 | 25.83 | 6.4 |
| 14.29 | 9.4 | 26.45 | 3.2 |
| 14.49 | 26.1 | 26.67 | 5.2 |
| 14.68 | 6.7 | 26.96 | 7.4 |
| 14.94 | 5.0 | 28.15 | 5.2 |
| 15.37 | 25.1 | 28.63 | 3.0 |
| 15.95 | 8.2 | 28.81 | 5.6 |
| 16.38 | 15.7 | 30.11 | 2.1 |
| 16.63 | 6.5 | 30.34 | 3.4 |
| 18.00 | 13.2 | 31.34 | 1.9 |
| 18.15 | 12.6 | 32.04 | 2.0 |
| 18.69 | 8.3 | 34.83 | 2.9 |
| 19.03 | 13.9 | 35.25 | 2.6 |
| 19.28 | 27.5 | 36.37 | 2.0 |
| 19.57 | 8.7 | | |
preferably, the crystal form C has X-ray powder diffraction intensities shown in Table 3;
preferably, the crystal form C has an X-ray powder diffraction pattern substantially as shown in FIG. 7;
preferably, the crystal form C exhibits endothermic peaks in a DSC analysis when heated to peak temperatures of about 160.99 °C and about 170.25 °C;
preferably, the crystal form C has a DSC profile substantially as shown in FIG. 8;
preferably, the crystal form C has a TGA profile substantially as shown in FIG. 9.

3. The polymorph according to claim 1, wherein the polymorph is a hydrate crystal form D of the compound of formula (I), wherein:
the hydrate crystal form D has an X-ray powder diffraction pattern comprising peaks at diffraction angles (2θ) of 7.71±0.2°, 10.96±0.2°, 12.34±0.2°, and 19.38±0.2°,
preferably further comprising peaks at diffraction angles (2θ) of 23.30±0.2°, 16.42±0.2°, 11.60±0.2°, 14.30±0.2°, and 3.81±0.2°, and
more preferably, further comprising peaks at diffraction angles (2θ) of 21.54±0.2°, 22.55±0.2°, 25.40±0.2°, 26.96±0.2°, 15.78±0.2°, and 21.29±0.2°;
preferably, the crystal form D has an X-ray powder diffraction pattern comprising diffraction angles (2θ) shown in Table 4, wherein the 2θ angles have a margin of error of ±0.20°:
**Table 4**
| 2θ (°) | Intensity% | 2θ (°) | Intensity% |
|---|---|---|---|
| 3.81 | 23.9 | 21.91 | 9.7 |
| 7.08 | 2.1 | 22.55 | 18.1 |
| 7.71 | 100.0 | 23.30 | 79.6 |
| 8.21 | 4.1 | 24.27 | 4.8 |
| 9.32 | 1.8 | 24.68 | 7.7 |
| 10.96 | 94.0 | 25.40 | 17.4 |
| 11.60 | 36 | 25.91 | 2.5 |
| 12.34 | 85.6 | 26.96 | 17.4 |
| 14.30 | 24.1 | 27.54 | 11.4 |
| 15.46 | 3.4 | 28.24 | 2.0 |
| 15.78 | 17.2 | 28.73 | 4.9 |
| 16.03 | 7.8 | 28.96 | 5.3 |
| 16.42 | 58.1 | 29.35 | 9.3 |
| 17.80 | 6.8 | 29.88 | 3.9 |
| 18.58 | 3.3 | 31.81 | 5.9 |
| 19.38 | 81.6 | 32.41 | 11.1 |
| 19.85 | 4.7 | 35.53 | 8.1 |
| 20.29 | 3.7 | 36.95 | 2.1 |
| 20.90 | 6.3 | 37.59 | 3.0 |
| 21.29 | 13.6 | 38.72 | 4.7 |
| 21.54 | 18.4 | | |
preferably, the crystal form D has X-ray powder diffraction intensities shown in Table 4;
preferably, the crystal form D has an X-ray powder diffraction pattern substantially as shown in FIG. 10;
preferably, the crystal form D exhibits endothermic peaks in a DSC analysis when heated to peak temperatures of about 62.09 °C, about 79.77 °C, and about 173.12 °C;
preferably, the crystal form D has a DSC profile substantially as shown in FIG. 11;
preferably, the crystal form D has a TGA profile substantially as shown in FIG. 12.

4. The polymorph according to claim 1, wherein the polymorph is a solvate crystal form of the compound of formula (I), including the following solvate crystal forms E, F, G, H, and I, wherein:
a trichloromethane solvate crystal form E has an X-ray powder diffraction pattern comprising peaks at diffraction angles (2θ) of 18.84±0.2°, 7.26±0.2°, 22.92±0.2°, and 9.35±0.2°,
preferably further comprising peaks at diffraction angles (2θ) of 22.05±0.2°, 18.36±0.2°, 6.21±0.2°, 3.60±0.2°, and 16.98±0.2°, and
more preferably, further comprising peaks at diffraction angles (2θ) of 12.50±0.2°, 34.99±0.2°, 16.11±0.2°, 20.51±0.2°, 19.57±0.2°, and 17.57±0.2°;
preferably, the crystal form E has an X-ray powder diffraction pattern comprising diffraction angles (2θ) shown in Table 5, wherein the 2θ angles have a margin of error of ±0.20°:
**Table 5**
| 2θ (°) | Intensity% | 2θ (°) | Intensity% |
|---|---|---|---|
| 3.60 | 21.9 | 20.51 | 13.9 |
| 6.21 | 34.6 | 20.92 | 3.3 |
| 7.26 | 78.8 | 22.05 | 37.7 |
| 9.35 | 55.3 | 22.92 | 59.1 |
| 10.12 | 1.7 | 23.53 | 4.8 |
| 11.04 | 2.0 | 23.95 | 1.9 |
| 12.50 | 16.4 | 24.36 | 4.4 |
| 14.18 | 4.4 | 24.81 | 2.1 |
| 15.11 | 1.6 | 25.23 | 2.1 |
| 15.66 | 3.7 | 26.12 | 1.8 |
| 16.11 | 14.2 | 26.94 | 1.7 |
| 16.61 | 6.3 | 28.48 | 5.4 |
| 16.98 | 20.7 | 29.09 | 2.7 |
| 17.57 | 9.6 | 29.37 | 2.0 |
| 18.01 | 1.9 | 29.64 | 1.7 |
| 18.36 | 35.2 | 31.69 | 4.8 |
| 18.84 | 100.0 | 32.27 | 2.0 |
| 19.57 | 11.4 | 33.41 | 7.6 |
| 19.90 | 7.1 | 34.99 | 15.3 |
preferably, the crystal form E has X-ray powder diffraction intensities shown in Table 5;
preferably, the crystal form E has an X-ray powder diffraction pattern substantially as shown in FIG. 13;
preferably, the crystal form E exhibits endothermic peaks in a DSC analysis when heated to peak temperatures of about 137.77 °C, about 159.79 °C, and about 175.44 °C;
preferably, the crystal form E has a DSC profile substantially as shown in FIG. 14;
preferably, the crystal form E has a TGA profile substantially as shown in FIG. 15;
a N-methylpyrrolidone solvate crystal form F has an X-ray powder diffraction pattern comprising peaks at diffraction angles (2θ) of 6.60±0.2°, 19.57±0.2°, 5.89±0.2°, and 14.96±0.2°,
preferably further comprising peaks at diffraction angles (2θ) of 25.69±0.2°, 3.27±0.2°, 17.49±0.2°, 19.32±0.2°, and 16.89±0.2°, and
more preferably, further comprising peaks at diffraction angles (2θ) of 16.01±0.2°, 18.07±0.2°, 21.13±0.2°, 22.46±0.2°, 15.48±0.2°, and 27.29±0.2°;
preferably, the crystal form F has an X-ray powder diffraction pattern comprising diffraction angles (2θ) shown in Table 6, wherein the 2θ angles have a margin of error of ±0.20°:
**Table 6**
| 2θ (°) | Intensity% | 2θ (°) | Intensity% |
|---|---|---|---|
| 3.27 | 34.5 | 21.83 | 8.5 |
| 5.89 | 37.2 | 22.24 | 15.3 |
| 6.60 | 100.0 | 22.46 | 19.8 |
| 7.24 | 9.7 | 23.24 | 13.0 |
| 8.86 | 15.5 | 24.03 | 9.2 |
| 9.10 | 3.9 | 24.61 | 8.5 |
| 9.66 | 13.8 | 25.01 | 14.4 |
| 11.82 | 11.8 | 25.69 | 35.5 |
| 12.15 | 12.6 | 27.29 | 17.6 |
| 13.28 | 16.4 | 27.54 | 14.4 |
| 14.64 | 5.3 | 28.90 | 3.0 |
| 14.96 | 36.2 | 29.35 | 3.7 |
| 15.48 | 19.6 | 29.35 | 3.7 |
| 16.01 | 25.8 | 29.97 | 3.6 |
| 16.89 | 28.0 | 30.25 | 7.8 |
| 17.49 | 34.3 | 31 | 4.9 |
| 18.07 | 24.4 | 31.30 | 3.6 |
| 18.52 | 11.8 | 32.82 | 4.0 |
| 19.32 | 32.8 | 33.05 | 5.2 |
| 19.57 | 38.1 | 33.37 | 3.0 |
| 20.18 | 4.5 | 33.76 | 5.2 |
| 21.13 | 23.3 | 37.25 | 2.4 |
| 21.42 | 12.5 | 39.32 | 2.7 |
preferably, the crystal form F has X-ray powder diffraction intensities shown in Table 6;
preferably, the crystal form F has an X-ray powder diffraction pattern substantially as shown in FIG. 16;
preferably, the crystal form F exhibits endothermic peaks in a DSC analysis when heated to peak temperatures of about 72.65 °C, about 95.65 °C, about 127.81 °C, and about 159.14 °C;
preferably, the crystal form F has a DSC profile substantially as shown in FIG. 17;
preferably, the crystal form F has a TGA profile substantially as shown in FIG. 18;
a N-methylpyrrolidone solvate crystal form G has an X-ray powder diffraction pattern comprising peaks at diffraction angles (2θ) of 3.50±0.2°, 6.97±0.2°, 13.91±0.2°, and 22.19±0.2°,
preferably further comprising peaks at diffraction angles (2θ) of 31.61±0.2°, 18.11±0.2°, 20.55±0.2°, 18.97±0.2°, and 15.76±0.2°, and
more preferably, further comprising peaks at diffraction angles (2θ) of 28.52±0.2°, 35.16±0.2°, 20.86±0.2°, 16.36±0.2°, 26.02±0.2°, and 17.18±0.2°;
preferably, the crystal form G has an X-ray powder diffraction pattern comprising diffraction angles (2θ) shown in Table 7, wherein the 2θ angles have a margin of error of ±0.20°:
**Table 7**
| 2θ(°) | Intensity% | 2θ(°) | Intensity% |
|---|---|---|---|
| 3.50 | 100.0 | 20.55 | 14.0 |
| 6.97 | 98.6 | 20.86 | 9.0 |
| 10.16 | 6.1 | 22.19 | 28.8 |
| 11.06 | 5.1 | 23.43 | 2.9 |
| 11.49 | 2.8 | 23.88 | 6.0 |
| 13.91 | 35.2 | 24.27 | 3.5 |
| 14.41 | 5.2 | 26.02 | 8.2 |
| 14.92 | 2.3 | 27.09 | 6.1 |
| 15.76 | 11.9 | 27.48 | 4.0 |
| 16.36 | 8.7 | 27.99 | 5.3 |
| 16.91 | 3.9 | 28.52 | 11.0 |
| 17.18 | 6.5 | 30.27 | 5.1 |
| 17.80 | 4.8 | 31.61 | 18.4 |
| 18.11 | 17.3 | 33.11 | 3.4 |
| 18.97 | 13.9 | 35.16 | 9.7 |
| 19.71 | 3.9 | 38.80 | 2.5 |
preferably, the crystal form G has X-ray powder diffraction intensities shown in Table 7;
preferably, the crystal form G has an X-ray powder diffraction pattern substantially as shown in FIG. 19;
preferably, the crystal form G exhibits endothermic peaks in a DSC analysis when heated to peak temperatures of about 109.95 °C and about 166.02 °C;
preferably, the crystal form G has a DSC profile substantially as shown in FIG. 20;
preferably, the crystal form G has a TGA profile substantially as shown in FIG. 21;
a tetrahydrofuran solvate crystal form H has an X-ray powder diffraction pattern comprising peaks at diffraction angles (2θ) of 3.29±0.2°, 3.71±0.2°, 19.71±0.2°, and 19.24±0.2°,
preferably further comprising peaks at diffraction angles (2θ) of 7.46±0.2°, 13.12±0.2°, 23.08±0.2°, 22.73±0.2°, and 6.60±0.2°, and
more preferably, further comprising peaks at diffraction angles (2θ) of 10.73±0.2°, 11.43±0.2°, 22.05±0.2°, 9.82±0.2°, 25.34±0.2°, and 16.83±0.2°;
preferably, the crystal form H has an X-ray powder diffraction pattern comprising diffraction angles (2θ) shown in Table 8, wherein the 2θ angles have a margin of error of ±0.20°:
**Table 8**
| 2θ (°) | Intensity% | 2θ (°) | Intensity% |
|---|---|---|---|
| 3.29 | 100.0 | 17.67 | 4.6 |
| 3.71 | 92.5 | 19.24 | 67.6 |
| 6.60 | 22.9 | 19.71 | 80.8 |
| 7.46 | 63.0 | 22.05 | 11.8 |
| 9.82 | 9.2 | 22.73 | 40.5 |
| 10.73 | 22.4 | 23.08 | 48.1 |
| 11.43 | 18.1 | 25.34 | 8.5 |
| 13.12 | 52.5 | 26.43 | 3.1 |
| 15.03 | 4.2 | 27.66 | 2.7 |
| 15.88 | 3.0 | 29.39 | 2.0 |
| 16.49 | 3.4 | 31.10 | 2.2 |
| 16.83 | 7.2 | 35.51 | 1.7 |
preferably, the crystal form H has X-ray powder diffraction intensities shown in Table 8;
preferably, the crystal form H has an X-ray powder diffraction pattern substantially as shown in FIG. 22;
preferably, the crystal form H exhibits endothermic peaks in a DSC analysis when heated to peak temperatures of about 82.80 °C and about 174.84 °C;
preferably, the crystal form H has a DSC profile substantially as shown in FIG. 23;
preferably, the crystal form H has a TGA profile substantially as shown in FIG. 24;
an ethanol solvate crystal form I has an X-ray powder diffraction pattern comprising peaks at diffraction angles (2θ) of 18.97±0.2°, 10.71±0.2°, 16.19±0.2°, and 22.67±0.2°,
preferably further comprising peaks at diffraction angles (2θ) of 12.79±0.2°, 15.13±0.2°, 17.99±0.2°, 22.52±0.2°, and 23.94±0.2°, and
more preferably, further comprising peaks at diffraction angles (2θ) of 16.53±0.2°, 24.99±0.2°, 21.85±0.2°, 19.28±0.2°, 26.69±0.2°, and 24.38±0.2°;
preferably, the crystal form I has an X-ray powder diffraction pattern comprising diffraction angles (2θ) shown in Table 9, wherein the 2θ angles have a margin of error of ±0.20°:
**Table 9**
| 2θ (°) | Intensity% | 2θ (°) | Intensity% |
|---|---|---|---|
| 5.30 | 6.6 | 22.67 | 31.8 |
| 6.36 | 1.4 | 22.87 | 13.3 |
| 6.94 | 12.2 | 23.16 | 5.0 |
| 8.77 | 4.6 | 23.46 | 5.5 |
| 10.71 | 92.6 | 23.94 | 16.6 |
| 11.25 | 3.3 | 24.38 | 13.6 |
| 11.77 | 1.8 | 24.99 | 15.4 |
| 12.38 | 1.6 | 25.71 | 8.7 |
| 12.79 | 29.5 | 25.94 | 4.5 |
| 13.24 | 8.7 | 26.31 | 4.6 |
| 13.92 | 5.5 | 26.69 | 14.1 |
| 14.20 | 3.9 | 27.16 | 4.0 |
| 14.56 | 10.8 | 27.83 | 6.9 |
| 15.13 | 25.0 | 28.44 | 4.8 |
| 15.40 | 4.5 | 28.75 | 2.6 |
| 16.19 | 35.0 | 29.13 | 2.2 |
| 16.53 | 15.4 | 29.92 | 3.2 |
| 17.08 | 2.1 | 30.34 | 2.6 |
| 17.35 | 2.5 | 31.12 | 1.3 |
| 17.61 | 6.7 | 31.38 | 1.9 |
| 17.99 | 22.5 | 31.95 | 3.9 |
| 18.97 | 100.0 | 32.29 | 1.7 |
| 19.28 | 14.1 | 32.88 | 2.5 |
| 19.87 | 12.6 | 33.36 | 1.6 |
| 20.20 | 13.1 | 33.74 | 1.2 |
| 20.57 | 2.9 | 34.98 | 1.8 |
| 21.07 | 12.2 | 35.92 | 1.9 |
| 21.27 | 9.4 | 36.42 | 1.9 |
| 21.70 | 9.5 | 37.31 | 1.4 |
| 21.85 | 14.6 | 38.45 | 1.3 |
| 22.52 | 19.7 | 39.21 | 1.9 |
preferably, the crystal form I has X-ray powder diffraction intensities shown in Table 9;
preferably, the crystal form I has an X-ray powder diffraction pattern substantially as shown in FIG. 25;
preferably, the crystal form I exhibits endothermic peaks in a DSC analysis when heated to peak temperatures of about 136.10 °C and about 160.74 °C;
preferably, the crystal form I has a DSC profile substantially as shown in FIG. 26;
preferably, the crystal form I has a TGA profile substantially as shown in FIG. 27.

5. The polymorph according to claim 1, wherein the polymorph is a metastable crystal form of the compound of formula (I), including the following crystal forms J, K, L, M, and N, wherein:
a metastable crystal form J has an X-ray powder diffraction pattern comprising peaks at diffraction angles (2θ) of 7.22±0.2°, 3.54±0.2°, 15.99±0.2°, and 19.34±0.2°,
preferably further comprising peaks at diffraction angles (2θ) of 22.77±0.2°, 18.33±0.2°, 17.80±0.2°, 10.93±0.2°, and 22.07±0.2°, and
more preferably, further comprising peaks at diffraction angles (2θ) of 33.38±0.2°, 27.77±0.2°, 29.53±0.2°, 9.95±0.2°, 20.16±0.2°, and 25.56±0.2°;
preferably, the crystal form J has an X-ray powder diffraction pattern comprising diffraction angles (2θ) shown in Table 10, wherein the 2θ angles have a margin of error of ±0.20°:
**Table 10**
| 2θ (°) | Intensity% | 2θ (°) | Intensity% |
|---|---|---|---|
| 3.54 | 63.8 | 21.30 | 2.1 |
| 7.22 | 100.0 | 22.07 | 5.9 |
| 9.95 | 3.6 | 22.77 | 11.3 |
| 10.93 | 6.7 | 23.63 | 2.0 |
| 12.95 | 1.9 | 24.20 | 2.1 |
| 14.61 | 1.7 | 24.79 | 2.8 |
| 14.86 | 2.4 | 25.56 | 2.9 |
| 15.99 | 20.5 | 27.77 | 4.5 |
| 16.81 | 2.6 | 28.85 | 1.3 |
| 17.80 | 9.0 | 29.53 | 4.0 |
| 18.33 | 10.3 | 30.91 | 2.2 |
| 19.34 | 15.6 | 33.38 | 5 |
| 20.16 | 3.6 | 39.45 | 1.0 |
preferably, the crystal form J has X-ray powder diffraction intensities shown in Table 10;
preferably, the crystal form J has an X-ray powder diffraction pattern substantially as shown in FIG. 28;
a metastable crystal form K has an X-ray powder diffraction pattern comprising peaks at diffraction angles (2θ) of 6.13±0.2°, 7.19±0.2°, 3.56±0.2°, and 22.63±0.2°,
preferably further comprising peaks at diffraction angles (2θ) of 18.56±0.2°, 12.34±0.2°, 16.83±0.2°, 9.31±0.2°, and 26.20±0.2°, and
more preferably, further comprising peaks at diffraction angles (2θ) of 19.63±0.2°, 20.26±0.2°, 23.72±0.2°, 17.40±0.2°, 34.48±0.2°, and 24.21±0.2°;
preferably, the crystal form K has an X-ray powder diffraction pattern comprising diffraction angles (2θ) shown in Table 11, wherein the 2θ angles have a margin of error of ±0.20°:
**Table 11**
| 2θ (°) | Intensity% | 2θ (°) | Intensity% |
|---|---|---|---|
| 3.56 | 35.1 | 21.68 | 6.1 |
| 6.13 | 100.0 | 22.00 | 3.1 |
| 7.19 | 39.3 | 22.63 | 31.4 |
| 9.31 | 12.1 | 23.20 | 2.5 |
| 11.51 | 2.2 | 23.72 | 8.4 |
| 12.34 | 17.9 | 24.21 | 7.8 |
| 14.06 | 5.0 | 24.99 | 3.0 |
| 14.53 | 4.9 | 26.20 | 10.1 |
| 15.62 | 5.6 | 28.48 | 5.9 |
| 16.83 | 17.6 | 29.53 | 3.4 |
| 17.40 | 8.3 | 30.11 | 2.7 |
| 17.79 | 2.6 | 31.26 | 7.4 |
| 18.56 | 21.7 | 31.94 | 2.0 |
| 19.12 | 3.8 | 34.48 | 8.1 |
| 19.63 | 9.9 | 35.53 | 2.3 |
| 20.26 | 8.5 | 38.75 | 1.9 |
| 20.82 | 7.7 | | |
preferably, the crystal form K has X-ray powder diffraction intensities shown in Table 11;
preferably, the crystal form K has an X-ray powder diffraction pattern substantially as shown in FIG. 29;
a metastable crystal form L has an X-ray powder diffraction pattern comprising peaks at diffraction angles (2θ) of 8.04±0.2°, 14.49±0.2°, 19.75±0.2°, and 15.95±0.2°,
preferably further comprising peaks at diffraction angles (2θ) of 18.70±0.2°, 20.85±0.2°, 4.00±0.2°, 23.27±0.2°, and 12.40±0.2°, and
more preferably, further comprising peaks at diffraction angles (2θ) of 16.22±0.2°, 15.58±0.2°, 14.27±0.2°, 12.13±0.2°, 15.43±0.2°, and 17.69±0.2°;
preferably, the crystal form L has an X-ray powder diffraction pattern comprising diffraction angles (2θ) shown in Table 12, wherein the 2θ angles have a margin of error of ±0.20°:
**Table 12**
| 2θ (°) | Intensity% | 2θ (°) | Intensity% |
|---|---|---|---|
| 4.00 | 29.7 | 21.54 | 16.0 |
| 8.04 | 100.0 | 22.78 | 6.9 |
| 9.02 | 9.9 | 23.27 | 29.4 |
| 9.29 | 8.1 | 23.63 | 9.6 |
| 10.84 | 4.8 | 24.36 | 17.2 |
| 12.13 | 18.7 | 24.89 | 7.2 |
| 12.40 | 25.8 | 25.46 | 6.2 |
| 13.79 | 13.3 | 26.10 | 3.2 |
| 14.27 | 20.0 | 26.96 | 5.9 |
| 14.49 | 77.0 | 28.07 | 7.2 |
| 15.43 | 18.5 | 28.51 | 5.3 |
| 15.58 | 20.7 | 28.86 | 2.5 |
| 15.95 | 47.5 | 29.69 | 2.8 |
| 16.22 | 24.6 | 31.43 | 5.4 |
| 16.84 | 3.9 | 31.82 | 2.7 |
| 17.39 | 3.9 | 32.08 | 3.9 |
| 17.69 | 17.2 | 32.35 | 2.4 |
| 18.17 | 14.6 | 35.24 | 4.4 |
| 18.70 | 47.5 | 36.07 | 2.4 |
| 19.75 | 63.9 | 37.30 | 1.9 |
| 20.31 | 8.5 | 38.44 | 2.3 |
| 20.85 | 37.2 | | |
preferably, the crystal form L has X-ray powder diffraction intensities shown in Table 12;
preferably, the crystal form L has an X-ray powder diffraction pattern substantially as shown in FIG. 30;
a metastable crystal form M has an X-ray powder diffraction pattern comprising peaks at diffraction angles (2θ) of 19.96±0.2°, 12.72±0.2°, 7.92±0.2°, and 11.12±0.2°,
preferably further comprising peaks at diffraction angles (2θ) of 16.54±0.2°, 3.95±0.2°, 21.66±0.2°, 24.01±0.2°, and 22.69±0.2°, and
more preferably, further comprising peaks at diffraction angles (2θ) of 14.45±0.2°, 11.92±0.2°, 15.93±0.2°, 20.98±0.2°, 23.59±0.2°, and 6.61±0.2°;
preferably, the crystal form M has an X-ray powder diffraction pattern comprising diffraction angles (2θ) shown in Table 13, wherein the 2θ angles have a margin of error of ±0.20°:
**Table 13**
| 2θ (°) | Intensity% | 2θ (°) | Intensity% |
|---|---|---|---|
| 3.95 | 55.2 | 22.69 | 27.5 |
| 6.61 | 7.2 | 23.10 | 2.8 |
| 7.92 | 67.6 | 23.59 | 8.3 |
| 9.49 | 3.7 | 24.01 | 35.2 |
| 9.98 | 2.2 | 24.69 | 3.9 |
| 11.12 | 65.9 | 26.12 | 2.9 |
| 11.92 | 20.5 | 26.49 | 3.7 |
| 12.72 | 84.2 | 26.72 | 6.1 |
| 13.84 | 2.5 | 27.33 | 2.8 |
| 14.45 | 26.4 | 27.79 | 6.1 |
| 14.80 | 2.5 | 29.12 | 3.7 |
| 15.93 | 10.3 | 30.22 | 5.2 |
| 16.54 | 56.6 | 32.74 | 3.9 |
| 17.77 | 2.8 | 32.96 | 4.5 |
| 18.21 | 7.1 | 33.17 | 4.0 |
| 19.96 | 100.0 | 36.42 | 2.1 |
| 20.98 | 8.8 | 36.78 | 2.8 |
| 21.66 | 42.7 | 39.08 | 1.7 |
preferably, the crystal form M has X-ray powder diffraction intensities shown in Table 13;
preferably, the crystal form M has an X-ray powder diffraction pattern substantially as shown in FIG. 31;
a metastable crystal form N has an X-ray powder diffraction pattern comprising peaks at diffraction angles (2θ) of 10.92±0.2°, 15.37±0.2°, 19.34±0.2°, and 20.46±0.2°,
preferably further comprising peaks at diffraction angles (2θ) of 12.95±0.2°, 6.93±0.2°, 18.04±0.2°, 16.43±0.2°, and 25.26±0.2°, and
more preferably, further comprising peaks at diffraction angles (2θ) of 5.43±0.2°, 10.75±0.2°, 14.20±0.2°, 23.12±0.2°, 17.87±0.2°, and 14.94±0.2°;
preferably, the crystal form N has an X-ray powder diffraction pattern comprising diffraction angles (2θ) shown in Table 14, wherein the 2θ angles have a margin of error of ±0.20°:
**Table 14**
| 2θ (°) | Intensity% | 2θ (°) | Intensity% |
|---|---|---|---|
| 5.43 | 13.3 | 23.70 | 2.4 |
| 6.93 | 17.2 | 23.93 | 1.2 |
| 7.42 | 2.0 | 24.23 | 5.6 |
| 8.85 | 9.7 | 24.50 | 5.3 |
| 10.75 | 12.7 | 25.26 | 14.4 |
| 10.92 | 100 | 25.83 | 3.7 |
| 12.95 | 21.6 | 26.10 | 1.5 |
| 14.20 | 12.0 | 26.47 | 2.7 |
| 14.71 | 1.4 | 26.78 | 4.6 |
| 14.94 | 10.1 | 27.09 | 2.9 |
| 15.37 | 48.2 | 27.66 | 1.7 |
| 16.20 | 10.1 | 28.17 | 2.0 |
| 16.43 | 15.4 | 28.63 | 6.6 |
| 16.68 | 3.8 | 28.88 | 2.6 |
| 17.87 | 11.0 | 29.67 | 1.3 |
| 18.04 | 15.7 | 30.29 | 4.0 |
| 19.07 | 9.7 | 31.32 | 1.9 |
| 19.34 | 27.8 | 32.33 | 1.6 |
| 19.59 | 8.2 | 32.72 | 2.2 |
| 20.04 | 3.7 | 33.00 | 1.4 |
| 20.46 | 23.1 | 34.36 | 1.9 |
| 21.15 | 8.7 | 34.82 | 1.2 |
| 21.63 | 8.4 | 35.26 | 2.1 |
| 22.01 | 7.4 | 35.98 | 1.8 |
| 22.65 | 3.8 | 36.29 | 1.5 |
| 23.12 | 12.0 | 39.17 | 3.6 |
| 23.42 | 3.0 | | |
preferably, the crystal form N has X-ray powder diffraction intensities shown in Table 14;
preferably, the crystal form N has an X-ray powder diffraction pattern substantially as shown in FIG. 32.

6. A method for preparing the polymorph according to any one of claims 1-5, comprising:
step 1: dissolving or dispersing compound I in a solvent; and
step 2: stirring at 0-50 °C for crystallization, or adding an antisolvent into a clarified solution of the compound for crystallization, or slowly evaporating the clarified solution of the compound.

7. The method according to claim 6, wherein the solvent is water, an organic solvent, or a mixed solvent thereof; the organic solvent is selected from alcohols, chloroalkanes, ketones, ethers, cyclic ethers, esters, alkanes, cycloalkanes, benzenoids, amides, sulfoxides, and a mixture thereof; preferably, the organic solvent is selected from methanol, ethanol, n-propanol, isopropanol, n-butanol, trifluoroethanol, acetonitrile, acetone, methyl ethyl ketone, methyl isobutyl ketone, 1,4-dioxane, tetrahydrofuran, 2-methyltetrahydrofuran, N,N-dimethylformamide, N,N-dimethylacetamide, *N-*methylpyrrolidone, dimethylsulfoxide, ethyl acetate, isopropyl acetate, dichloromethane, trichloromethane, trichloroethane, tetrachloromethane, methyl *tert-*butyl ether, cyclopentyl methyl ether, 2-methoxyethyl ether, isopropyl ether, diethyl ether, n-heptane, n-hexane, isooctane, pentane, cyclohexane, cyclopentane, methylcyclohexane, benzene, toluene, xylene, and a mixture thereof.

8. A pharmaceutical composition, comprising at least one of the polymorphs according to any one of claims 1-5 and a pharmaceutically acceptable carrier.

9. Use of the polymorph according to any one of claims 1-5 for manufacturing a medicament for the treatment of a metabolic disease, a tumor, an autoimmune disease, or a metastatic disease.

10. The use according to claim 9, wherein the metabolic disease, the tumor, the autoimmune disease, or the metastatic disease is selected from T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, and polycystic ovary syndrome.

11. The use according to claim 9, wherein the metabolic disease, the tumor, the autoimmune disease, or the metastatic disease is selected from T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, obesity, eating disorders, weight gain caused by use of other medicaments, excessive sugar craving, dyslipidemia, and hyperinsulinemia.
